# EUROPEAN PATENT APPLICATION

(11) **EP 1 099 760 A1**
(43) Date of publication of application: **16.05.2001**
(21) Application number: 99203723.4
(22) Date of filing: 09.11.1999
(51) Int. Cl.: C12N 15/32, C12N 15/62, C07K 14/325, A01H 5/00, A01N 63/00

(54) **Bacillus thuringiensis Cry1Ia-Cry1Ba hybrid toxins**

(71) Applicant: CENTRUM VOOR PLANTENVEREDELINGS- EN REPRODUKTIEONDERZOEK (CPRO), 6708 PB Wageningen (NL)
(72) Inventor: de Maagd, Rudolf Aart, 6708 HL Wageningen (NL); Bosch, Hendrik Jan, 3572 ZM Utrecht (NL)
(74) Representative: De Hoop, Eric

(57) **Abstract**

*Bacillus thuringiensis* hybrid toxin fragment comprising structural domains I, II and III in this order starting from the N-terminal, wherein the domains are derived from at least two different Cry proteins, domain I is domain I of any *Bacillus thuringiensis* Cry protein or a part of said domain or a peptide substantially similar to said domain, domain II is domain II of Cry1la or a part of said domain or a peptide substantially similar to said domain, and domain III is domain III of Cry1Ba or a part of said domain or a peptide substantially similar domain.

SEQUENCE LISTING

<110> CPRO-DLO

<120> Bacillus thuringiensis hybrid toxins

<130> E158870

<140>
<141>

<160> 10

<170> PatentIn Ver. 2.1

<210> 1
<211> 3651
<212> DNA
<213> Artificial Sequence

<220>
<223> Description of Artificial Sequence: hybrid
      protoxin gene SN15

<400> 1
atgaaactaa agaatcaaga taagcatcaa agtttttcta gcaatgcgaa agtagataaa 60
atctctacgg attcactaaa aaatgaaaca gatatagaat tacaaaacat taatcatgaa 120
gattgtttga aaatgtctga gtatgaaaat gtagagccgt ttgttagtgc atcaacaatt 180
caaacaggta ttggtattgc gggtaaaata cttggtaccc taggcgttcc ttttgcagga 240
caagtagcta gtctttatag ttttatctta ggtgagctat ggcctaaggg gaaaaatcaa 300
tgggaaatct ttatggaaca tgtagaagag attattaatc aaaaaatatc aacttatgca 360
agaaataaag cacttacaga cttgaaagga ttaggagatg ccttagctgt ctaccatgat 420
tcgcttgaaa gttgggttgg aaatcgtaat aacacaaggg ctaggagtgt tgtcaagagc 480
caatatatcg cattagaatt gatgttcgtt cagaaactac cttcttttgc agtgtctgga 540
gaggaggtac cattattacc gatatatgcc caagctgcaa atttacattt gttgctatta 600
agagatgcat ctatttttgg aaaagagtgg ggattatcat cttcagaaat ttcaacattt 660
tataaccgtc aagtcgaacg agcaggagat tattcctacc attgtgtgaa atggtatagc 720
acaggtctaa ataacttgag gggtacaaat gccgaaagtt gggtacgata taatcaattc 780
cgtagagaca tgactttaat ggtactagat ttagtggcac tatttccaag ctatgataca 840
caaatgtatc caattaaaac tacagcccaa cttacaagag aagtatatac agacgcaatt 900
gggacagtac atccgcatcc aagttttaca agtacgactt ggtataataa taatgcacct 960
tcgttctctg ccatagaggc tgctgttgtt cgaaacccgc atctactcga ttttctagaa 1020
caagttacaa tttacagctt attaagtcga tggagtaaca ctcagtatat gaatatgtgg 1080
ggaggacata aactagaatt ccgaacaata ggaggaacgt taaatatctc aacacaagga 1140
tctactaata cttctattaa tcctgtaaca ttaccgttca cttctcgaga cgtctatagg 1200
actgaatcat tggcagggct gaatctattt ttaactcaac ctgttaatgg agtacctagg 1260
gttgattttc attggaaatt cgtcacacat ccgatcgcat ctgataattt ctattatcca 1320
gggtatgctg gaattgggac gcaattacag gattcagaaa atgaattacc acctgaagca 1380

acaggacagc caaattatga atcttatagt catagattat ctcatatagg actcatttca 1440
gcatcacatg tgaaagcatc ggtatattct tggacgcatc gtagtgcgga ccgtacgaat 1500
acgattggac caaatagaat cacccaaatc ccaatggtaa aagcatccga acttcctcaa 1560
ggtaccactg ttgttagagg accaggattt actggtgggg atattcttcg aagaacgaat 1620
actggtggat ttggaccgat aagagtaact gttaacggac cattaacaca aagatatcgt 1680
ataggattcc gctatgcttc aactgtagat tttgatttct ttgtatcacg tggaggtact 1740
actgtaaata attttagatt cctacgtaca atgaacagtg gagacgaact aaaatacgga 1800
aattttgtga gacgtgcttt tactacacct tttactttta cacaaattca agatataatt 1860
cgaacgtcta ttcaaggcct tagtggaaat ggggaagtgt atatagataa aattgaaatt 1920
attccagtta ctgcaacctt cgaagcagaa tatgatttag aaagagcgca agaggcggtg 1980
aatgctctgt ttactaatac gaatccaaga agattgaaaa cagatgtgac agattatcat 2040
attgatcaag tatccaattt agtggattgt ttatcagatg aattttgtct ggatgaaaag 2100
cgagaattgt ccgagaaagt caaacatgcg aagcgactca gtgatgagcg gaatttactt 2160
caagatccaa acttcagagg gatcaataga caaccagacc gtggctggag aggaagtaca 2220
gatattacca tccaaggagg agatgacgta ttcaaagaga attacgtcac actaccgggt 2280
accgttgatg agtgctatcc aacgtattta tatcagaaaa tagatgagtc gaaattaaaa 2340
gcttataccc gttatgaatt aagagggtat atcgaagata gtcaagactt agaaatctat 2400
ttgatccgtt acaatgcaaa acacgaaata gtaaatgtgc caggcacggg ttccttatgg 2460
ccgctttcag cccaaagtcc aatcggaaag tgtggagaac cgaatcgatg cgcgccacac 2520
cttgaatgga atcctgatct agattgttcc tgcagagacg gggaaaaatg tgcacatcat 2580
tcccatcatt tcaccttgga tattgatgtt ggatgtacag acttaaatga ggacttaggt 2640
gtatgggtga tattcaagat taagacgcaa gatggccatg caagactagg gaatctagag 2700
tttctcgaag agaaaccatt attaggggaa gcactagctc gtgtgaaaag agcggagaag 2760
aagtggagag acaaacgaga gaaactgcag ttggaaacaa atattgttta taaagaggca 2820
aaagaatctg tagatgcttt atttgtaaac tctcaatatg atagattaca agtggatacg 2880
aacatcgcga tgattcatgc ggcagataaa cgcgttcata gaatccggga agcgtatctg 2940
ccagagttgt ctgtgattcc aggtgtcaat gcggccattt tcgaagaatt agagggacgt 3000
atttttacag cgtattcctt atatgatgcg agaaatgtca ttaaaaatgg cgatttcaat 3060
aatggcttat tatgctggaa cgtgaaaggt catgtagatg tagaagagca aaacaaccac 3120
cgttcggtcc ttgttatccc agaatgggag gcagaagtgt cacaagaggt tcgtgtctgt 3180
ccaggtcgtg gctatatcct tcgtgtcaca gcatataaag agggatatgg agagggctgc 3240
gtaacgatcc atgagatcga agacaataca gacgaactga aattcagcaa ctgtgtagaa 3300
gaggaagtat atccaaacaa cacagtaacg tgtaataatt atactgggac tcaagaagaa 3360
tatgagggta cgtacacttc tcgtaatcaa ggatatgacg aagcctatgg taataaccct 3420
tccgtaccag ctgattacgc ttcagtctat gaagaaaaat cgtatacaga tggacgaaga 3480
gagaatcctt gtgaatctaa cagaggctat ggggattaca caccactacc ggctggttat 3540
gtaacaaagg atttagagta cttcccagag accgataagg tatggattga gatcggagaa 3600
acagaaggaa cattcatcgt ggatagcgtg gaattactcc ttatggagga a 3651

<210> 2
<211> 1217
<212> PRT
<213> Artificial Sequence

<220>
<223> Description of Artificial Sequence: protein
      encoded by the gene SN15

<400> 2
Met Lys Leu Lys Asn Gln Asp Lys His Gln Ser Phe Ser Ser Asn Ala
1               5                   10                  15

Lys Val Asp Lys Ile Ser Thr Asp Ser Leu Lys Asn Glu Thr Asp Ile
            20                  25                  30

Glu Leu Gln Asn Ile Asn His Glu Asp Cys Leu Lys Met Ser Glu Tyr
        35                  40                  45

Glu Asn Val Glu Pro Phe Val Ser Ala Ser Thr Ile Gln Thr Gly Ile
    50                  55                  60

Gly Ile Ala Gly Lys Ile Leu Gly Thr Leu Gly Val Pro Phe Ala Gly
65                  70                  75                  80

Gln Val Ala Ser Leu Tyr Ser Phe Ile Leu Gly Glu Leu Trp Pro Lys
                85                  90                  95

Gly Lys Asn Gln Trp Glu Ile Phe Met Glu His Val Glu Glu Ile Ile
            100                 105                 110

Asn Gln Lys Ile Ser Thr Tyr Ala Arg Asn Lys Ala Leu Thr Asp Leu
        115                 120                 125

Lys Gly Leu Gly Asp Ala Leu Ala Val Tyr His Asp Ser Leu Glu Ser
    130                 135                 140

Trp Val Gly Asn Arg Asn Asn Thr Arg Ala Arg Ser Val Val Lys Ser
145                 150                 155                 160

Gln Tyr Ile Ala Leu Glu Leu Met Phe Val Gln Lys Leu Pro Ser Phe
                165                 170                 175

Ala Val Ser Gly Glu Glu Val Pro Leu Leu Pro Ile Tyr Ala Gln Ala
            180                 185                 190

Ala Asn Leu His Leu Leu Leu Leu Arg Asp Ala Ser Ile Phe Gly Lys
        195                 200                 205

Glu Trp Gly Leu Ser Ser Ser Glu Ile Ser Thr Phe Tyr Asn Arg Gln
    210                 215                 220

Val Glu Arg Ala Gly Asp Tyr Ser Tyr His Cys Val Lys Trp Tyr Ser
225                 230                 235                 240

Thr Gly Leu Asn Asn Leu Arg Gly Thr Asn Ala Glu Ser Trp Val Arg

                245                 250                 255

Tyr Asn Gln Phe Arg Arg Asp Met Thr Leu Met Val Leu Asp Leu Val
            260                 265                 270

Ala Leu Phe Pro Ser Tyr Asp Thr Gln Met Tyr Pro Ile Lys Thr Thr
        275                 280                 285

Ala Gln Leu Thr Arg Glu Val Tyr Thr Asp Ala Ile Gly Thr Val His
    290                 295                 300

Pro His Pro Ser Phe Thr Ser Thr Thr Trp Tyr Asn Asn Asn Ala Pro
305                 310                 315                 320

Ser Phe Ser Ala Ile Glu Ala Ala Val Val Arg Asn Pro His Leu Leu
                325                 330                 335

Asp Phe Leu Glu Gln Val Thr Ile Tyr Ser Leu Leu Ser Arg Trp Ser
            340                 345                 350

Asn Thr Gln Tyr Met Asn Met Trp Gly Gly His Lys Leu Glu Phe Arg
        355                 360                 365

Thr Ile Gly Gly Thr Leu Asn Ile Ser Thr Gln Gly Ser Thr Asn Thr
    370                 375                 380

Ser Ile Asn Pro Val Thr Leu Pro Phe Thr Ser Arg Asp Val Tyr Arg
385                 390                 395                 400

Thr Glu Ser Leu Ala Gly Leu Asn Leu Phe Leu Thr Gln Pro Val Asn
                405                 410                 415

Gly Val Pro Arg Val Asp Phe His Trp Lys Phe Val Thr His Pro Ile
            420                 425                 430

Ala Ser Asp Asn Phe Tyr Tyr Pro Gly Tyr Ala Gly Ile Gly Thr Gln
        435                 440                 445

Leu Gln Asp Ser Glu Asn Glu Leu Pro Pro Glu Ala Thr Gly Gln Pro
    450                 455                 460

Asn Tyr Glu Ser Tyr Ser His Arg Leu Ser His Ile Gly Leu Ile Ser
465                 470                 475                 480

Ala Ser His Val Lys Ala Ser Val Tyr Ser Trp Thr His Arg Ser Ala
                485                 490                 495

Asp Arg Thr Asn Thr Ile Gly Pro Asn Arg Ile Thr Gln Ile Pro Met

500                 505                 510

Val Lys Ala Ser Glu Leu Pro Gln Gly Thr Thr Val Val Arg Gly Pro
        515                 520                 525

Gly Phe Thr Gly Gly Asp Ile Leu Arg Arg Thr Asn Thr Gly Gly Phe
    530                 535                 540

Gly Pro Ile Arg Val Thr Val Asn Gly Pro Leu Thr Gln Arg Tyr Arg
545                 550                 555                 560

Ile Gly Phe Arg Tyr Ala Ser Thr Val Asp Phe Asp Phe Phe Val Ser
                565                 570                 575

Arg Gly Gly Thr Thr Val Asn Asn Phe Arg Phe Leu Arg Thr Met Asn
            580                 585                 590

Ser Gly Asp Glu Leu Lys Tyr Gly Asn Phe Val Arg Arg Ala Phe Thr
        595                 600                 605

Thr Pro Phe Thr Phe Thr Gln Ile Gln Asp Ile Ile Arg Thr Ser Ile
    610                 615                 620

Gln Gly Leu Ser Gly Asn Gly Glu Val Tyr Ile Asp Lys Ile Glu Ile
625                 630                 635                 640

Ile Pro Val Thr Ala Thr Phe Glu Ala Glu Tyr Asp Leu Glu Arg Ala
                645                 650                 655

Gln Glu Ala Val Asn Ala Leu Phe Thr Asn Thr Asn Pro Arg Arg Leu
            660                 665                 670

Lys Thr Asp Val Thr Asp Tyr His Ile Asp Gln Val Ser Asn Leu Val
        675                 680                 685

Asp Cys Leu Ser Asp Glu Phe Cys Leu Asp Glu Lys Arg Glu Leu Ser
    690                 695                 700

Glu Lys Val Lys His Ala Lys Arg Leu Ser Asp Glu Arg Asn Leu Leu
705                 710                 715                 720

Gln Asp Pro Asn Phe Arg Gly Ile Asn Arg Gln Pro Asp Arg Gly Trp
                725                 730                 735

Arg Gly Ser Thr Asp Ile Thr Ile Gln Gly Gly Asp Asp Val Phe Lys
            740                 745                 750

Glu Asn Tyr Val Thr Leu Pro Gly Thr Val Asp Glu Cys Tyr Pro Thr

        755                 760                 765

Tyr Leu Tyr Gln Lys Ile Asp Glu Ser Lys Leu Lys Ala Tyr Thr Arg
    770                 775                 780

Tyr Glu Leu Arg Gly Tyr Ile Glu Asp Ser Gln Asp Leu Glu Ile Tyr
785                 790                 795                 800

Leu Ile Arg Tyr Asn Ala Lys His Glu Ile Val Asn Val Pro Gly Thr
                805                 810                 815

Gly Ser Leu Trp Pro Leu Ser Ala Gln Ser Pro Ile Gly Lys Cys Gly
            820                 825                 830

Glu Pro Asn Arg Cys Ala Pro His Leu Glu Trp Asn Pro Asp Leu Asp
        835                 840                 845

Cys Ser Cys Arg Asp Gly Glu Lys Cys Ala His His Ser His His Phe
    850                 855                 860

Thr Leu Asp Ile Asp Val Gly Cys Thr Asp Leu Asn Glu Asp Leu Gly
865                 870                 875                 880

Val Trp Val Ile Phe Lys Ile Lys Thr Gln Asp Gly His Ala Arg Leu
                885                 890                 895

Gly Asn Leu Glu Phe Leu Glu Glu Lys Pro Leu Leu Gly Glu Ala Leu
            900                 905                 910

Ala Arg Val Lys Arg Ala Glu Lys Lys Trp Arg Asp Lys Arg Glu Lys
        915                 920                 925

Leu Gln Leu Glu Thr Asn Ile Val Tyr Lys Glu Ala Lys Glu Ser Val
    930                 935                 940

Asp Ala Leu Phe Val Asn Ser Gln Tyr Asp Arg Leu Gln Val Asp Thr
945                 950                 955                 960

Asn Ile Ala Met Ile His Ala Ala Asp Lys Arg Val His Arg Ile Arg
                965                 970                 975

Glu Ala Tyr Leu Pro Glu Leu Ser Val Ile Pro Gly Val Asn Ala Ala
            980                 985                 990

Ile Phe Glu Glu Leu Glu Gly Arg Ile Phe Thr Ala Tyr Ser Leu Tyr
        995                 1000                1005

Asp Ala Arg Asn Val Ile Lys Asn Gly Asp Phe Asn Asn Gly Leu Leu

1010                1015                1020

Cys Trp Asn Val Lys Gly His Val Asp Val Glu Glu Gln Asn Asn His
1025                1030                1035                1040

Arg Ser Val Leu Val Ile Pro Glu Trp Glu Ala Glu Val Ser Gln Glu
                1045                1050                1055

Val Arg Val Cys Pro Gly Arg Gly Tyr Ile Leu Arg Val Thr Ala Tyr
            1060                1065                1070

Lys Glu Gly Tyr Gly Glu Gly Cys Val Thr Ile His Glu Ile Glu Asp
        1075                1080                1085

Asn Thr Asp Glu Leu Lys Phe Ser Asn Cys Val Glu Glu Glu Val Tyr
    1090                1095                1100

Pro Asn Asn Thr Val Thr Cys Asn Asn Tyr Thr Gly Thr Gln Glu Glu
1105                1110                1115                1120

Tyr Glu Gly Thr Tyr Thr Ser Arg Asn Gln Gly Tyr Asp Glu Ala Tyr
                1125                1130                1135

Gly Asn Asn Pro Ser Val Pro Ala Asp Tyr Ala Ser Val Tyr Glu Glu
            1140                1145                1150

Lys Ser Tyr Thr Asp Gly Arg Arg Glu Asn Pro Cys Glu Ser Asn Arg
        1155                1160                1165

Gly Tyr Gly Asp Tyr Thr Pro Leu Pro Ala Gly Tyr Val Thr Lys Asp
    1170                1175                1180

Leu Glu Tyr Phe Pro Glu Thr Asp Lys Val Trp Ile Glu Ile Gly Glu
1185                1190                1195                1200

Thr Glu Gly Thr Phe Ile Val Asp Ser Val Glu Leu Leu Leu Met Glu
                1205                1210                1215

Glu

<210> 3
<211> 3624
<212> DNA
<213> Artificial Sequence

<220>
<223> Description of Artificial Sequence: hybrid
      protoxin gene SN19

<400> 3
atggcttcaa ataggaaaaa tgagaatgaa attataaatg ctgtatcgaa tcattccgca 60
caaatggatc tattaccaga tgctcgtatt gaggatagct tgtgtatagc cgaggggaac 120
aatatcgatc catttgttag cgcatcaaca gtccaaacgg gtattaacat agctggtaga 180
atactaggcg tattgggcgt accgtttgct ggacaactag ctagttttta tagttttctt 240
gttggtgaat tatggccccg cggcagagat cagtgggaaa ttttcctaga acatgtcgaa 300
caacttataa atcaacaaat aacagaaaat gctaggaata cggctcttgc tcgattacaa 360
ggtttaggag attccttcag agcctatcaa cagtcacttg aagattggct agaaaaccgt 420
gatgatgcaa gaacgagaag tgttctttat acccaatata tagctttaga acttgatttt 480
cttaatgcga tgccgctttt cgcaattaga aaccaagaag ttccattatt gatggtatat 540
gctcaagctg caaatttaca cctattatta ttgagagatg cctctctttt tggtagtgaa 600
tttgggctta catcgcagga aattcaacgc tattatgagc gccaagtgga acgaacgaga 660
gattattccg actattgcgt agaatggtat aatacaggtc taaatagctt gagagggaca 720
aatgccgcaa gttgggtacg gtataatcaa ttccgtagag atctaacgtt aggagtatta 780
gatctagtgg cactattccc aagctatgac actcgcactt atccaataaa tacgagtgct 840
cagttaacaa gagaagttta tacagacgca attgggacag tacatccgca tccaagtttt 900
acaagtacga cttggtataa taataatgca ccttcgttct ctgccataga ggctgctgtt 960
gttcgaaacc cgcatctact cgattttcta gaacaagtta caatttacag cttattaagt 1020
cgatggagta acactcagta tatgaatatg tggggaggac ataaactaga attccgaaca 1080
ataggaggaa cgttaaatat ctcaacacaa ggatctacta atacttctat taatcctgta 1140
acattaccgt tcacttctcg agacgtctat aggactgaat cattggcagg gctgaatcta 1200
tttttaactc aacctgttaa tggagtacct agggttgatt ttcattggaa attcgtcaca 1260
catccgatcg catctgataa tttctattat ccagggtatg ctggaattgg gacgcaatta 1320
caggattcag aaaatgaatt accacctgaa gcaacaggac agccaaatta tgaatcttat 1380
agtcatagat tatctcatat aggactcatt tcagcatcac atgtgaaagc atcggtatat 1440
tcttggacgc atcgtagtgc ggaccgtacg aatacgattg gaccaaatag aatcacccaa 1500
atcccaatgg taaaagcatc cgaacttcct caaggtacca ctgttgttag aggaccagga 1560
tttactggtg ggatattctt tcgaagaacg aatactggtg gatttggacc gataagagta 1620
actgttaacg gaccattaac acaaagatat cgtataggat tccgctatgc ttcaactgta 1680
gattttgatt tctttgtatc acgtggaggt actactgtaa ataattttag attcctacgt 1740
acaatgaaca gtggagacga actaaaatac ggaaattttg tgagacgtgc ttttactaca 1800
ccttttactt ttacacaaat tcaagatata attcgaacgt ctattcaagg ccttagtgga 1860
aatggggaag tgtatataga taaaattgaa attattccag ttactgcaac cttcgaagca 1920
gaatatgatt tagaaagagc gcaagaggcg gtgaatgctc tgtttactaa tacgaatcca 1980
agaagattga aaacagatgt gacagattat catattgatc aagtatccaa tttagtggat 2040
tgtttatcag atgaattttg tctggatgaa aagcgagaat tgtccgagaa agtcaaacat 2100
gcgaagcgac tcagtgatga gcggaattta cttcaagatc caaacttcag agggatcaat 2160
agacaaccag accgtggctg gagaggaagt acagatatta ccatccaagg aggagatgac 2220
gtattcaaag agaattacgt cacactaccg ggtaccgttg atgagtgcta tccaacgtat 2280
ttatatcaga aaatagatga gtcgaaatta aaagcttata cccgttatga attaagaggg 2340
tatatcgaag atagtcaaga cttagaaatc tatttgatcc gttacaatgc aaaacacgaa 2400
atagtaaatg tgccaggcac gggttcctta tggccgcttt cagcccaaag tccaatcgga 2460
aagtgtggag aaccgaatcg atgcgcgcca caccttgaat ggaatcctga tctagattgt 2520
tcctgcagag acggggaaaa atgtgcacat cattcccatc atttcacctt ggatattgat 2580

gttggatgta cagacttaaa tgaggactta ggtgtatggg tgatattcaa gattaagacg 2640
caagatggcc atgcaagact agggaatcta gagtttctcg aagagaaacc attattaggg 2700
gaagcactag ctcgtgtgaa aagagcggag aagaagtgga gagacaaacg agagaaactg 2760
cagttggaaa caaatattgt ttataaagag gcaaaagaat ctgtagatgc tttatttgta 2820
aactctcaat atgatagatt acaagtggat acgaacatcg cgatgattca tgcggcagat 2880
aaacgcgttc atagaatccg ggaagcgtat ctgccagagt tgtctgtgat tccaggtgtc 2940
aatgcggcca ttttcgaaga attagaggga cgtattttta cagcgtattc cttatatgat 3000
gcgagaaatg tcattaaaaa tggcgatttc aataatggct tattatgctg gaacgtgaaa 3060
ggtcatgtag atgtagaaga gcaaaacaac caccgttcgg tccttgttat cccagaatgg 3120
gaggcagaag tgtcacaaga ggttcgtgtc tgtccaggtc gtggctatat ccttcgtgtc 3180
acagcatata aagagggata tggagagggc tgcgtaacga tccatgagat cgaagacaat 3240
acagacgaac tgaaattcag caactgtgta gaagaggaag tatatccaaa caacacagta 3300
acgtgtaata attatactgg gactcaagaa gaatatgagg gtacgtacac ttctcgtaat 3360
caaggatatg acgaagccta tggtaataac ccttccgtac cagctgatta cgcttcagtc 3420
tatgaagaaa aatcgtatac agatggacga agagagaatc cttgtgaatc taacagaggc 3480
tatggggatt acacaccact accggctggt tatgtaacaa aggatttaga gtacttccca 3540
gagaccgata aggtatggat tgagatcgga gaaacagaag gaacattcat cgtggatagc 3600
gtggaattac tccttatgga ggaa 3624

<210> 4
<211> 1208
<212> PRT
<213> Artificial Sequence

<220>
<223> Description of Artificial Sequence: protein
      encoded by the gene SN19

<400> 4
Met Ala Ser Asn Arg Lys Asn Glu Asn Glu Ile Ile Asn Ala Val Ser
1               5                   10                  15

Asn His Ser Ala Gln Met Asp Leu Leu Pro Asp Ala Arg Ile Glu Asp
            20                  25                  30

Ser Leu Cys Ile Ala Glu Gly Asn Asn Ile Asp Pro Phe Val Ser Ala
        35                  40                  45

Ser Thr Val Gln Thr Gly Ile Asn Ile Ala Gly Arg Ile Leu Gly Val
    50                  55                  60

Leu Gly Val Pro Phe Ala Gly Gln Leu Ala Ser Phe Tyr Ser Phe Leu
65                  70                  75                  80

Val Gly Glu Leu Trp Pro Arg Gly Arg Asp Gln Trp Glu Ile Phe Leu
                85                  90                  95

Glu His Val Glu Gln Leu Ile Asn Gln Gln Ile Thr Glu Asn Ala Arg
            100                 105                 110

Asn Thr Ala Leu Ala Arg Leu Gln Gly Leu Gly Asp Ser Phe Arg Ala
        115                 120                 125

Tyr Gln Gln Ser Leu Glu Asp Trp Leu Glu Asn Arg Asp Asp Ala Arg
    130                 135                 140

Thr Arg Ser Val Leu Tyr Thr Gln Tyr Ile Ala Leu Glu Leu Asp Phe
145                 150                 155                 160

Leu Asn Ala Met Pro Leu Phe Ala Ile Arg Asn Gln Glu Val Pro Leu
                165                 170                 175

Leu Met Val Tyr Ala Gln Ala Ala Asn Leu His Leu Leu Leu Leu Arg
            180                 185                 190

Asp Ala Ser Leu Phe Gly Ser Glu Phe Gly Leu Thr Ser Gln Glu Ile
        195                 200                 205

Gln Arg Tyr Tyr Glu Arg Gln Val Glu Arg Thr Arg Asp Tyr Ser Asp
    210                 215                 220

Tyr Cys Val Glu Trp Tyr Asn Thr Gly Leu Asn Ser Leu Arg Gly Thr
225                 230                 235                 240

Asn Ala Ala Ser Trp Val Arg Tyr Asn Gln Phe Arg Arg Asp Leu Thr
                245                 250                 255

Leu Gly Val Leu Asp Leu Val Ala Leu Phe Pro Ser Tyr Asp Thr Arg
            260                 265                 270

Thr Tyr Pro Ile Asn Thr Ser Ala Gln Leu Thr Arg Glu Val Tyr Thr
        275                 280                 285

Asp Ala Ile Gly Thr Val His Pro His Pro Ser Phe Thr Ser Thr Thr
    290                 295                 300

Trp Tyr Asn Asn Asn Ala Pro Ser Phe Ser Ala Ile Glu Ala Ala Val
305                 310                 315                 320

Val Arg Asn Pro His Leu Leu Asp Phe Leu Glu Gln Val Thr Ile Tyr
                325                 330                 335

Ser Leu Leu Ser Arg Trp Ser Asn Thr Gln Tyr Met Asn Met Trp Gly
            340                 345                 350

Gly His Lys Leu Glu Phe Arg Thr Ile Gly Gly Thr Leu Asn Ile Ser
        355                 360                 365

Thr Gln Gly Ser Thr Asn Thr Ser Ile Asn Pro Val Thr Leu Pro Phe
    370                 375                 380

Thr Ser Arg Asp Val Tyr Arg Thr Glu Ser Leu Ala Gly Leu Asn Leu
385                 390                 395                 400

Phe Leu Thr Gln Pro Val Asn Gly Val Pro Arg Val Asp Phe His Trp
                405                 410                 415

Lys Phe Val Thr His Pro Ile Ala Ser Asp Asn Phe Tyr Tyr Pro Gly
            420                 425                 430

Tyr Ala Gly Ile Gly Thr Gln Leu Gln Asp Ser Glu Asn Glu Leu Pro
        435                 440                 445

Pro Glu Ala Thr Gly Gln Pro Asn Tyr Glu Ser Tyr Ser His Arg Leu
    450                 455                 460

Ser His Ile Gly Leu Ile Ser Ala Ser His Val Lys Ala Ser Val Tyr
465                 470                 475                 480

Ser Trp Thr His Arg Ser Ala Asp Arg Thr Asn Thr Ile Gly Pro Asn
                485                 490                 495

Arg Ile Thr Gln Ile Pro Met Val Lys Ala Ser Glu Leu Pro Gln Gly
            500                 505                 510

Thr Thr Val Val Arg Gly Pro Gly Phe Thr Gly Gly Asp Ile Leu Arg
        515                 520                 525

Arg Thr Asn Thr Gly Gly Phe Gly Pro Ile Arg Val Thr Val Asn Gly
    530                 535                 540

Pro Leu Thr Gln Arg Tyr Arg Ile Gly Phe Arg Tyr Ala Ser Thr Val
545                 550                 555                 560

Asp Phe Asp Phe Phe Val Ser Arg Gly Gly Thr Thr Val Asn Asn Phe
                565                 570                 575

Arg Phe Leu Arg Thr Met Asn Ser Gly Asp Glu Leu Lys Tyr Gly Asn
            580                 585                 590

Phe Val Arg Arg Ala Phe Thr Thr Pro Phe Thr Phe Thr Gln Ile Gln
        595                 600                 605

Asp Ile Ile Arg Thr Ser Ile Gln Gly Leu Ser Gly Asn Gly Glu Val
    610                 615                 620

Tyr Ile Asp Lys Ile Glu Ile Ile Pro Val Thr Ala Thr Phe Glu Ala
625                 630                 635                 640

Glu Tyr Asp Leu Glu Arg Ala Gln Glu Ala Val Asn Ala Leu Phe Thr
                645                 650                 655

Asn Thr Asn Pro Arg Arg Leu Lys Thr Asp Val Thr Asp Tyr His Ile
            660                 665                 670

Asp Gln Val Ser Asn Leu Val Asp Cys Leu Ser Asp Glu Phe Cys Leu
        675                 680                 685

Asp Glu Lys Arg Glu Leu Ser Glu Lys Val Lys His Ala Lys Arg Leu
    690                 695                 700

Ser Asp Glu Arg Asn Leu Leu Gln Asp Pro Asn Phe Arg Gly Ile Asn
705                 710                 715                 720

Arg Gln Pro Asp Arg Gly Trp Arg Gly Ser Thr Asp Ile Thr Ile Gln
                725                 730                 735

Gly Gly Asp Asp Val Phe Lys Glu Asn Tyr Val Thr Leu Pro Gly Thr
            740                 745                 750

Val Asp Glu Cys Tyr Pro Thr Tyr Leu Tyr Gln Lys Ile Asp Glu Ser
        755                 760                 765

Lys Leu Lys Ala Tyr Thr Arg Tyr Glu Leu Arg Gly Tyr Ile Glu Asp
    770                 775                 780

Ser Gln Asp Leu Glu Ile Tyr Leu Ile Arg Tyr Asn Ala Lys His Glu
785                 790                 795                 800

Ile Val Asn Val Pro Gly Thr Gly Ser Leu Trp Pro Leu Ser Ala Gln
                805                 810                 815

Ser Pro Ile Gly Lys Cys Gly Glu Pro Asn Arg Cys Ala Pro His Leu
            820                 825                 830

Glu Trp Asn Pro Asp Leu Asp Cys Ser Cys Arg Asp Gly Glu Lys Cys
        835                 840                 845

Ala His His Ser His His Phe Thr Leu Asp Ile Asp Val Gly Cys Thr
    850                 855                 860

Asp Leu Asn Glu Asp Leu Gly Val Trp Val Ile Phe Lys Ile Lys Thr
865                 870                 875                 880

Gln Asp Gly His Ala Arg Leu Gly Asn Leu Glu Phe Leu Glu Glu Lys
                885                 890                 895

Pro Leu Leu Gly Glu Ala Leu Ala Arg Val Lys Arg Ala Glu Lys Lys
            900                 905                 910

Trp Arg Asp Lys Arg Glu Lys Leu Gln Leu Glu Thr Asn Ile Val Tyr
        915                 920                 925

Lys Glu Ala Lys Glu Ser Val Asp Ala Leu Phe Val Asn Ser Gln Tyr
    930                 935                 940

Asp Arg Leu Gln Val Asp Thr Asn Ile Ala Met Ile His Ala Ala Asp
945                 950                 955                 960

Lys Arg Val His Arg Ile Arg Glu Ala Tyr Leu Pro Glu Leu Ser Val
                965                 970                 975

Ile Pro Gly Val Asn Ala Ala Ile Phe Glu Glu Leu Glu Gly Arg Ile
            980                 985                 990

Phe Thr Ala Tyr Ser Leu Tyr Asp Ala Arg Asn Val Ile Lys Asn Gly
        995                 1000                1005

Asp Phe Asn Asn Gly Leu Leu Cys Trp Asn Val Lys Gly His Val Asp
    1010                1015                1020

Val Glu Glu Gln Asn Asn His Arg Ser Val Leu Val Ile Pro Glu Trp
1025                1030                1035                1040

Glu Ala Glu Val Ser Gln Glu Val Arg Val Cys Pro Gly Arg Gly Tyr
                1045                1050                1055

Ile Leu Arg Val Thr Ala Tyr Lys Glu Gly Tyr Gly Glu Gly Cys Val
            1060                1065                1070

Thr Ile His Glu Ile Glu Asp Asn Thr Asp Glu Leu Lys Phe Ser Asn
        1075                1080                1085

Cys Val Glu Glu Glu Val Tyr Pro Asn Asn Thr Val Thr Cys Asn Asn
    1090                1095                1100

Tyr Thr Gly Thr Gln Glu Glu Tyr Glu Gly Thr Tyr Thr Ser Arg Asn
1105                1110                1115                1120

Gln Gly Tyr Asp Glu Ala Tyr Gly Asn Asn Pro Ser Val Pro Ala Asp
            1125                1130                1135

Tyr Ala Ser Val Tyr Glu Glu Lys Ser Tyr Thr Asp Gly Arg Arg Glu
        1140                1145                1150

Asn Pro Cys Glu Ser Asn Arg Gly Tyr Gly Asp Tyr Thr Pro Leu Pro
    1155                1160                1165

Ala Gly Tyr Val Thr Lys Asp Leu Glu Tyr Phe Pro Glu Thr Asp Lys
  1170                1175                1180

Val Trp Ile Glu Ile Gly Glu Thr Glu Gly Thr Phe Ile Val Asp Ser
1185                1190                1195                1200

Val Glu Leu Leu Leu Met Glu Glu
            1205

<210> 5
<211> 3627
<212> DNA
<213> Artificial Sequence

<220>
<223> Description of Artificial Sequence: modified
      cry1Ba gene

<400> 5
atgacttcaa ataggaaaaa tgagaatgaa attataaatg ctgtatcgaa tcattccgca 60
caaatggatc tattaccaga tgctcgtatt gaggatagct tgtgtatagc cgaggggaac 120
aatatcgatc catttgttag cgcatcaaca gtccaaacgg gtattaacat agctggtaga 180
atactaggcg tattgggcgt accgtttgct ggacaactag ctagttttta tagttttctt 240
gttggtgaat tatggccccg cggcagagat cagtgggaaa ttttcctaga acatgtcgaa 300
caacttataa atcaacaaat aacagaaaat gctaggaata cggctcttgc tcgattacaa 360
ggtttaggag attccttcag agcctatcaa cagtcacttg aagattggct agaaaaccgt 420
gatgatgcaa gaacgagaag tgttctttat acccaatata tagctttaga acttgatttt 480
cttaatgcga tgccgctttt cgcaattaga aaccaagaag ttccattatt gatggtatat 540
gctcaagctg caaatttaca cctattatta ttgagagatg cctctctttt tggtagtgaa 600
tttgggctta catcgcagga aattcaacgc tattatgagc gccaagtgga acgaacgaga 660
gattattccg actattgcgt agaatggtat aatacaggtc taaatagctt gagagggaca 720
aatgccgcaa gttgggtacg gtataatcaa ttccgtagag atctaacgtt aggagtatta 780
gatctagtgg cactattccc aagctatgac actcgcactt atccaataaa tacgagtgct 840
cagttaacaa gagaagttta tacagacgca attggagcaa caggggtaaa tatggcaagt 900
atgaattggt ataataataa tgcaccttcg ttctctgcca tagaggctgc ggctatccga 960
agcccgcatc tacttgattt tctagaacaa cttacaattt ttagcgcttc atcacgatgg 1020
agtaatacta ggcatatgac ttattggcgg gggcacacga ttcaatctcg gccaatagga 1080

ggcggattaa atacctcaac gcatggggct accaatactt ctattaatcc tgtaacatta 1140
cggttcgcat ctcgagacgt ttataggact gaatcatatg caggagtgct tctatgggga 1200
atttaccttg aacctattca tggtgtccct actgttaggt ttaattttac gaaccctcag 1260
aatatttctg atagaggtac cgctaactat agtcaacctt atgagtcacc tgggcttcaa 1320
ttaaaagatt cagaaactga attaccacca gaaacaacag aacgaccaaa ttatgaatct 1380
tacagtcaca ggttatctca tataggtata attttacaat ccagggtgaa tgtaccggta 1440
tattcttgga cgcatcgtag tgcggaccgt acgaatacga ttggaccaaa tagaatcacc 1500
caaatcccaa tggtaaaagc atccgaactt cctcaaggta ccactgttgt tagaggacca 1560
ggatttactg gtggggatat tcttcgaaga acgaatactg gtggatttgg accgataaga 1620
gtaactgtta acggaccatt aacacaaaga tatcgtatag gattccgcta tgcttcaact 1680
gtagattttg atttctttgt atcacgtgga ggtactactg taaataattt tagattccta 1740
cgtacaatga acagtggaga cgaactaaaa tacggaaatt ttgtgagacg tgcttttact 1800
acacctttta cttttacaca aattcaagat ataattcgaa cgtctattca aggccttagt 1860
ggaaatgggg aagtgtatat agataaaatt gaaattattc cagttactgc aaccttcgaa 1920
gcagaatatg atttagaaag agcgcaagag gcggtgaatg ctctgtttac taatacgaat 1980
ccaagaagat tgaaaacaga tgtgacagat tatcatattg atcaagtatc caatttagtg 2040
gattgtttat cagatgaatt ttgtctggat gaaaagcgag aattgtccga gaaagtcaaa 2100
catgcgaagc gactcagtga tgagcggaat ttacttcaag atccaaactt cagagggatc 2160
aatagacaac cagaccgtgg ctggagagga agtacagata ttaccatcca aggaggagat 2220
gacgtattca aagagaatta cgtcacacta ccgggtaccg ttgatgagtg ctatccaacg 2280
tatttatatc agaaaataga tgagtcgaaa ttaaaagctt atacccgtta tgaattaaga 2340
gggtatatcg aagatagtca agacttagaa atctatttga tccgttacaa tgcaaaacac 2400
gaaatagtaa atgtgccagg cacgggttcc ttatggccgc tttcagccca aagtccaatc 2460
ggaaagtgtg gagaaccgaa tcgatgcgcg ccacaccttg aatggaatcc tgatctagat 2520
tgttcctgca gagacgggga aaaatgtgca catcattccc atcatttcac cttggatatt 2580
gatgttggat gtacagactt aaatgaggac ttaggtgtat gggtgatatt caagattaag 2640
acgcaagatg gccatgcaag actagggaat ctagagtttc tcgaagagaa accattatta 2700
ggggaagcac tagctcgtgt gaaaagagcg gagaagaagt ggagagacaa acgagagaaa 2760
ctgcagttgg aaacaaatat tgtttataaa gaggcaaaag aatctgtaga tgctttattt 2820
gtaaactctc aatatgatag attacaagtg gatacgaaca tcgcgatgat tcatgcggca 2880
gataaacgcg ttcatagaat ccgggaagcg tatctgccag agttgtctgt gattccaggt 2940
gtcaatgcgg ccattttcga agaattagag ggacgtattt ttacagcgta ttccttatat 3000
gatgcgagaa atgtcattaa aaatggcgat ttcaataatg gcttattatg ctggaacgtg 3060
aaaggtcatg tagatgtaga agagcaaaac aaccaccgtt cggtccttgt tatcccagaa 3120
tgggaggcag aagtgtcaca agaggttcgt gtctgtccag gtcgtggcta tatccttcgt 3180
gtcacagcat ataaagaggg atatggagag ggctgcgtaa cgatccatga gatcgaagac 3240
aatacagacg aactgaaatt cagcaactgt gtagaagagg aagtatatcc aaacaacaca 3300
gtaacgtgta ataattatac tgggactcaa gaagaatatg agggtacgta cacttctcgt 3360
aatcaaggat atgacgaagc ctatggtaat aacccttccg taccagctga ttacgcttca 3420
gtctatgaag aaaaatcgta tacagatgga cgaagagaga atccttgtga atctaacaga 3480
ggctatgggg attacacacc actaccggct ggttatgtaa caaaggattt agagtacttc 3540
ccagagaccg ataaggtatg gattgagatc ggagaaacag aaggaacatt catcgtggat 3600
agcgtggaat tactccttat ggaggaa 3627

<210> 6
<211> 1209
<212> PRT

<213> Artificial Sequence

<220>
<223> Description of Artificial Sequence: protein
      encoded by the modified cry1Ba gene

<400> 6
Met Thr Ser Asn Arg Lys Asn Glu Asn Glu Ile Ile Asn Ala Val Ser
  1               5                  10                  15

Asn His Ser Ala Gln Met Asp Leu Leu Pro Asp Ala Arg Ile Glu Asp
             20                  25                  30

Ser Leu Cys Ile Ala Glu Gly Asn Asn Ile Asp Pro Phe Val Ser Ala
         35                  40                  45

Ser Thr Val Gln Thr Gly Ile Asn Ile Ala Gly Arg Ile Leu Gly Val
     50                  55                  60

Leu Gly Val Pro Phe Ala Gly Gln Leu Ala Ser Phe Tyr Ser Phe Leu
 65                  70                  75                  80

Val Gly Glu Leu Trp Pro Arg Gly Arg Asp Gln Trp Glu Ile Phe Leu
                 85                  90                  95

Glu His Val Glu Gln Leu Ile Asn Gln Gln Ile Thr Glu Asn Ala Arg
            100                 105                 110

Asn Thr Ala Leu Ala Arg Leu Gln Gly Leu Gly Asp Ser Phe Arg Ala
        115                 120                 125

Tyr Gln Gln Ser Leu Glu Asp Trp Leu Glu Asn Arg Asp Asp Ala Arg
    130                 135                 140

Thr Arg Ser Val Leu Tyr Thr Gln Tyr Ile Ala Leu Glu Leu Asp Phe
145                 150                 155                 160

Leu Asn Ala Met Pro Leu Phe Ala Ile Arg Asn Gln Glu Val Pro Leu
                165                 170                 175

Leu Met Val Tyr Ala Gln Ala Ala Asn Leu His Leu Leu Leu Leu Arg
            180                 185                 190

Asp Ala Ser Leu Phe Gly Ser Glu Phe Gly Leu Thr Ser Gln Glu Ile
        195                 200                 205

Gln Arg Tyr Tyr Glu Arg Gln Val Glu Arg Thr Arg Asp Tyr Ser Asp
    210                 215                 220

Tyr Cys Val Glu Trp Tyr Asn Thr Gly Leu Asn Ser Leu Arg Gly Thr
225                 230                 235                 240

Asn Ala Ala Ser Trp Val Arg Tyr Asn Gln Phe Arg Arg Asp Leu Thr
                245                 250                 255

Leu Gly Val Leu Asp Leu Val Ala Leu Phe Pro Ser Tyr Asp Thr Arg
            260                 265                 270

Thr Tyr Pro Ile Asn Thr Ser Ala Gln Leu Thr Arg Glu Val Tyr Thr
        275                 280                 285

Asp Ala Ile Gly Ala Thr Gly Val Asn Met Ala Ser Met Asn Trp Tyr
    290                 295                 300

Asn Asn Asn Ala Pro Ser Phe Ser Ala Ile Glu Ala Ala Ala Ile Arg
305                 310                 315                 320

Ser Pro His Leu Leu Asp Phe Leu Glu Gln Leu Thr Ile Phe Ser Ala
                325                 330                 335

Ser Ser Arg Trp Ser Asn Thr Arg His Met Thr Tyr Trp Arg Gly His
            340                 345                 350

Thr Ile Gln Ser Arg Pro Ile Gly Gly Gly Leu Asn Thr Ser Thr His
        355                 360                 365

Gly Ala Thr Asn Thr Ser Ile Asn Pro Val Thr Leu Arg Phe Ala Ser
    370                 375                 380

Arg Asp Val Tyr Arg Thr Glu Ser Tyr Ala Gly Val Leu Leu Trp Gly
385                 390                 395                 400

Ile Tyr Leu Glu Pro Ile His Gly Val Pro Thr Val Arg Phe Asn Phe
                405                 410                 415

Thr Asn Pro Gln Asn Ile Ser Asp Arg Gly Thr Ala Asn Tyr Ser Gln
            420                 425                 430

Pro Tyr Glu Ser Pro Gly Leu Gln Leu Lys Asp Ser Glu Thr Glu Leu
        435                 440                 445

Pro Pro Glu Thr Thr Glu Arg Pro Asn Tyr Glu Ser Tyr Ser His Arg
    450                 455                 460

Leu Ser His Ile Gly Ile Ile Leu Gln Ser Arg Val Asn Val Pro Val
465                 470                 475                 480

Tyr Ser Trp Thr His Arg Ser Ala Asp Arg Thr Asn Thr Ile Gly Pro
                485                 490                 495

Asn Arg Ile Thr Gln Ile Pro Met Val Lys Ala Ser Glu Leu Pro Gln
            500                 505                 510

Gly Thr Thr Val Val Arg Gly Pro Gly Phe Thr Gly Gly Asp Ile Leu
        515                 520                 525

Arg Arg Thr Asn Thr Gly Gly Phe Gly Pro Ile Arg Val Thr Val Asn
    530                 535                 540

Gly Pro Leu Thr Gln Arg Tyr Arg Ile Gly Phe Arg Tyr Ala Ser Thr
545                 550                 555                 560

Val Asp Phe Asp Phe Phe Val Ser Arg Gly Gly Thr Thr Val Asn Asn
                565                 570                 575

Phe Arg Phe Leu Arg Thr Met Asn Ser Gly Asp Glu Leu Lys Tyr Gly
            580                 585                 590

Asn Phe Val Arg Arg Ala Phe Thr Thr Pro Phe Thr Phe Thr Gln Ile
        595                 600                 605

Gln Asp Ile Ile Arg Thr Ser Ile Gln Gly Leu Ser Gly Asn Gly Glu
    610                 615                 620

Val Tyr Ile Asp Lys Ile Glu Ile Ile Pro Val Thr Ala Thr Phe Glu
625                 630                 635                 640

Ala Glu Tyr Asp Leu Glu Arg Ala Gln Glu Ala Val Asn Ala Leu Phe
                645                 650                 655

Thr Asn Thr Asn Pro Arg Arg Leu Lys Thr Asp Val Thr Asp Tyr His
            660                 665                 670

Ile Asp Gln Val Ser Asn Leu Val Asp Cys Leu Ser Asp Glu Phe Cys
        675                 680                 685

Leu Asp Glu Lys Arg Glu Leu Ser Glu Lys Val Lys His Ala Lys Arg
    690                 695                 700

Leu Ser Asp Glu Arg Asn Leu Leu Gln Asp Pro Asn Phe Arg Gly Ile
705                 710                 715                 720

Asn Arg Gln Pro Asp Arg Gly Trp Arg Gly Ser Thr Asp Ile Thr Ile
                725                 730                 735

Gln Gly Gly Asp Asp Val Phe Lys Glu Asn Tyr Val Thr Leu Pro Gly
            740                 745                 750

Thr Val Asp Glu Cys Tyr Pro Thr Tyr Leu Tyr Gln Lys Ile Asp Glu
        755                 760                 765

Ser Lys Leu Lys Ala Tyr Thr Arg Tyr Glu Leu Arg Gly Tyr Ile Glu
    770                 775                 780

Asp Ser Gln Asp Leu Glu Ile Tyr Leu Ile Arg Tyr Asn Ala Lys His
785                 790                 795                 800

Glu Ile Val Asn Val Pro Gly Thr Gly Ser Leu Trp Pro Leu Ser Ala
                805                 810                 815

Gln Ser Pro Ile Gly Lys Cys Gly Glu Pro Asn Arg Cys Ala Pro His
            820                 825                 830

Leu Glu Trp Asn Pro Asp Leu Asp Cys Ser Cys Arg Asp Gly Glu Lys
        835                 840                 845

Cys Ala His His Ser His His Phe Thr Leu Asp Ile Asp Val Gly Cys
    850                 855                 860

Thr Asp Leu Asn Glu Asp Leu Gly Val Trp Val Ile Phe Lys Ile Lys
865                 870                 875                 880

Thr Gln Asp Gly His Ala Arg Leu Gly Asn Leu Glu Phe Leu Glu Glu
                885                 890                 895

Lys Pro Leu Leu Gly Glu Ala Leu Ala Arg Val Lys Arg Ala Glu Lys
            900                 905                 910

Lys Trp Arg Asp Lys Arg Glu Lys Leu Gln Leu Glu Thr Asn Ile Val
        915                 920                 925

Tyr Lys Glu Ala Lys Glu Ser Val Asp Ala Leu Phe Val Asn Ser Gln
    930                 935                 940

Tyr Asp Arg Leu Gln Val Asp Thr Asn Ile Ala Met Ile His Ala Ala
945                 950                 955                 960

Asp Lys Arg Val His Arg Ile Arg Glu Ala Tyr Leu Pro Glu Leu Ser
                965                 970                 975

Val Ile Pro Gly Val Asn Ala Ala Ile Phe Glu Glu Leu Glu Gly Arg
            980                 985                 990

Ile Phe Thr Ala Tyr Ser Leu Tyr Asp Ala Arg Asn Val Ile Lys Asn
        995                 1000                1005

Gly Asp Phe Asn Asn Gly Leu Leu Cys Trp Asn Val Lys Gly His Val
    1010                1015                1020

Asp Val Glu Glu Gln Asn Asn His Arg Ser Val Leu Val Ile Pro Glu
1025                1030                1035                1040

Trp Glu Ala Glu Val Ser Gln Glu Val Arg Val Cys Pro Gly Arg Gly
                1045                1050                1055

Tyr Ile Leu Arg Val Thr Ala Tyr Lys Glu Gly Tyr Gly Glu Gly Cys
            1060                1065                1070

Val Thr Ile His Glu Ile Glu Asp Asn Thr Asp Glu Leu Lys Phe Ser
        1075                1080                1085

Asn Cys Val Glu Glu Glu Val Tyr Pro Asn Asn Thr Val Thr Cys Asn
    1090                1095                1100

Asn Tyr Thr Gly Thr Gln Glu Glu Tyr Glu Gly Thr Tyr Thr Ser Arg
1105                1110                1115                1120

Asn Gln Gly Tyr Asp Glu Ala Tyr Gly Asn Asn Pro Ser Val Pro Ala
                1125                1130                1135

Asp Tyr Ala Ser Val Tyr Glu Glu Lys Ser Tyr Thr Asp Gly Arg Arg
            1140                1145                1150

Glu Asn Pro Cys Glu Ser Asn Arg Gly Tyr Gly Asp Tyr Thr Pro Leu
        1155                1160                1165

Pro Ala Gly Tyr Val Thr Lys Asp Leu Glu Tyr Phe Pro Glu Thr Asp
    1170                1175                1180

Lys Val Trp Ile Glu Ile Gly Glu Thr Glu Gly Thr Phe Ile Val Asp
1185                1190                1195                1200

Ser Val Glu Leu Leu Leu Met Glu Glu
                1205

<210> 7
<211> 2160
<212> DNA

<213> Artificial Sequence

<220>
<223> Description of Artificial Sequence: modified
      cry1Ia gene

<400> 7
atgaaactaa agaatcaaga taagcatcaa agtttttcta gcaatgcgaa agtagataaa 60
atctctacgg attcactaaa aaatgaaaca gatatagaat tacaaaacat taatcatgaa 120
gattgtttga aaatgtctga gtatgaaaat gtagagccgt ttgttagtgc atcaacaatt 180
caaacaggta ttggtattgc gggtaaaata cttggtaccc taggcgttcc ttttgcagga 240
caagtagcta gtctttatag ttttatctta ggtgagctat ggcctaaggg gaaaaatcaa 300
tgggaaatct ttatggaaca tgtagaagag attattaatc aaaaaatatc aacttatgca 360
agaaataaag cacttacaga cttgaaagga ttaggagatg ccttagctgt ctaccatgat 420
tcgcttgaaa gttgggttgg aaatcgtaat aacacaaggg ctaggagtgt tgtcaagagc 480
caatatatcg cattagaatt gatgttcgtt cagaaactac cttcttttgc agtgtctgga 540
gaggaggtac cattattacc gatatatgcc caagctgcaa atttacattt gttgctatta 600
agagatgcat ctatttttgg aaaagagtgg ggattatcat cttcagaaat ttcaacattt 660
tataaccgtc aagtcgaacg agcaggagat tattcctacc attgtgtgaa atggtatagc 720
acaggtctaa ataacttgag gggtacaaat gccgaaagtt gggtacgata taatcaattc 780
cgtagagaca tgactttaat ggtactagat ttagtggcac tatttccaag ctatgataca 840
caaatgtatc caattaaaac tacagcccaa cttacaagag aagtatatac agacgcaatt 900
gggacagtac atccgcatcc aagttttaca agtacgactt ggtataataa taatgcacct 960
tcgttctctg ccatagaggc tgctgttgtt cgaaacccgc atctactcga ttttctagaa 1020
caagttacaa tttacagctt attaagtcga tggagtaaca ctcagtatat gaatatgtgg 1080
ggaggacata aactagaatt ccgaacaata ggaggaacgt taaatatctc aacacaagga 1140
tctactaata cttctattaa tcctgtaaca ttaccgttca cttctcgaga cgtctatagg 1200
actgaatcat tggcagggct gaatctattt ttaactcaac ctgttaatgg agtacctagg 1260
gttgattttc attggaaatt cgtcacacat ccgatcgcat ctgataattt ctattatcca 1320
gggtatgctg gaattgggac gcaattacag gattcagaaa atgaattacc acctgaagca 1380
acaggacagc caaattatga atcttatagt catagattat ctcatatagg actcatttca 1440
gcatcacatg tgaaagcatt ggtatattct tggacgcatc gtagtgcgga ccgtacaaat 1500
acaattgagc caaatagcat tacacaaata ccattagtaa aagctttcaa tctgtcttca 1560
ggtgccgctg tagtgagagg accaggattt acaggtgggg atatccttcg aagaacgaat 1620
actggtacat ttggggatat acgagtaaat attaatccac catttgcaca aagatatcgc 1680
gtgaggattc gctatgcttc taccacagat ttacaattcc atacgtcaat taacggtaaa 1740
gctattaatc aaggtaattt ttcagcaact atgaatagag gagaggactt agactataaa 1800
acctttagaa ctgtaggctt taccactcca tttagctttt tagatgtaca aagtacattc 1860
acaataggtg cttggaactt ctcttcaggt aacgaagttt atatagatag aattgaattt 1920
gttccggtag aagtaacata tgaggcagaa tatgattttg aaaaagcgca agagaaggtt 1980
actgcactgt ttacatctac gaatccaaga ggattaaaaa cagatgtaaa ggattatcat 2040
attgaccagg tatcaaattt agtagagtct ctatcagatg aattctatct tgatgaaaag 2100
agagaattat tcgagatagt taaatacgcg aagcaactcc atattgagcg taacatgtag 2160

<210> 8
<211> 719
<212> PRT

<213> Artificial Sequence

<220>
<223> Description of Artificial Sequence: protein
      encoded by the modified cry1Ia gene

<400> 8
Met Lys Leu Lys Asn Gln Asp Lys His Gln Ser Phe Ser Ser Asn Ala
  1               5                  10                  15

Lys Val Asp Lys Ile Ser Thr Asp Ser Leu Lys Asn Glu Thr Asp Ile
             20                  25                  30

Glu Leu Gln Asn Ile Asn His Glu Asp Cys Leu Lys Met Ser Glu Tyr
         35                  40                  45

Glu Asn Val Glu Pro Phe Val Ser Ala Ser Thr Ile Gln Thr Gly Ile
     50                  55                  60

Gly Ile Ala Gly Lys Ile Leu Gly Thr Leu Gly Val Pro Phe Ala Gly
 65                  70                  75                  80

Gln Val Ala Ser Leu Tyr Ser Phe Ile Leu Gly Glu Leu Trp Pro Lys
                 85                  90                  95

Gly Lys Asn Gln Trp Glu Ile Phe Met Glu His Val Glu Glu Ile Ile
            100                 105                 110

Asn Gln Lys Ile Ser Thr Tyr Ala Arg Asn Lys Ala Leu Thr Asp Leu
        115                 120                 125

Lys Gly Leu Gly Asp Ala Leu Ala Val Tyr His Asp Ser Leu Glu Ser
    130                 135                 140

Trp Val Gly Asn Arg Asn Asn Thr Arg Ala Arg Ser Val Val Lys Ser
145                 150                 155                 160

Gln Tyr Ile Ala Leu Glu Leu Met Phe Val Gln Lys Leu Pro Ser Phe
                165                 170                 175

Ala Val Ser Gly Glu Glu Val Pro Leu Leu Pro Ile Tyr Ala Gln Ala
            180                 185                 190

Ala Asn Leu His Leu Leu Leu Leu Arg Asp Ala Ser Ile Phe Gly Lys
        195                 200                 205

Glu Trp Gly Leu Ser Ser Ser Glu Ile Ser Thr Phe Tyr Asn Arg Gln
    210                 215                 220

Val Glu Arg Ala Gly Asp Tyr Ser Tyr His Cys Val Lys Trp Tyr Ser
225                 230                 235                 240

Thr Gly Leu Asn Asn Leu Arg Gly Thr Asn Ala Glu Ser Trp Val Arg
                245                 250                 255

Tyr Asn Gln Phe Arg Arg Asp Met Thr Leu Met Val Leu Asp Leu Val
            260                 265                 270

Ala Leu Phe Pro Ser Tyr Asp Thr Gln Met Tyr Pro Ile Lys Thr Thr
        275                 280                 285

Ala Gln Leu Thr Arg Glu Val Tyr Thr Asp Ala Ile Gly Thr Val His
    290                 295                 300

Pro His Pro Ser Phe Thr Ser Thr Thr Trp Tyr Asn Asn Asn Ala Pro
305                 310                 315                 320

Ser Phe Ser Ala Ile Glu Ala Ala Val Val Arg Asn Pro His Leu Leu
                325                 330                 335

Asp Phe Leu Glu Gln Val Thr Ile Tyr Ser Leu Leu Ser Arg Trp Ser
            340                 345                 350

Asn Thr Gln Tyr Met Asn Met Trp Gly Gly His Lys Leu Glu Phe Arg
        355                 360                 365

Thr Ile Gly Gly Thr Leu Asn Ile Ser Thr Gln Gly Ser Thr Asn Thr
    370                 375                 380

Ser Ile Asn Pro Val Thr Leu Pro Phe Thr Ser Arg Asp Val Tyr Arg
385                 390                 395                 400

Thr Glu Ser Leu Ala Gly Leu Asn Leu Phe Leu Thr Gln Pro Val Asn
                405                 410                 415

Gly Val Pro Arg Val Asp Phe His Trp Lys Phe Val Thr His Pro Ile
            420                 425                 430

Ala Ser Asp Asn Phe Tyr Tyr Pro Gly Tyr Ala Gly Ile Gly Thr Gln
        435                 440                 445

Leu Gln Asp Ser Glu Asn Glu Leu Pro Pro Glu Ala Thr Gly Gln Pro
    450                 455                 460

Asn Tyr Glu Ser Tyr Ser His Arg Leu Ser His Ile Gly Leu Ile Ser
465                 470                 475                 480

Ala Ser His Val Lys Ala Leu Val Tyr Ser Trp Thr His Arg Ser Ala
                485                 490                 495

Asp Arg Thr Asn Thr Ile Glu Pro Asn Ser Ile Thr Gln Ile Pro Leu
            500                 505                 510

Val Lys Ala Phe Asn Leu Ser Ser Gly Ala Ala Val Val Arg Gly Pro
        515                 520                 525

Gly Phe Thr Gly Gly Asp Ile Leu Arg Arg Thr Asn Thr Gly Thr Phe
    530                 535                 540

Gly Asp Ile Arg Val Asn Ile Asn Pro Pro Phe Ala Gln Arg Tyr Arg
545                 550                 555                 560

Val Arg Ile Arg Tyr Ala Ser Thr Thr Asp Leu Gln Phe His Thr Ser
                565                 570                 575

Ile Asn Gly Lys Ala Ile Asn Gln Gly Asn Phe Ser Ala Thr Met Asn
            580                 585                 590

Arg Gly Glu Asp Leu Asp Tyr Lys Thr Phe Arg Thr Val Gly Phe Thr
        595                 600                 605

Thr Pro Phe Ser Phe Leu Asp Val Gln Ser Thr Phe Thr Ile Gly Ala
    610                 615                 620

Trp Asn Phe Ser Ser Gly Asn Glu Val Tyr Ile Asp Arg Ile Glu Phe
625                 630                 635                 640

Val Pro Val Glu Val Thr Tyr Glu Ala Glu Tyr Asp Phe Glu Lys Ala
                645                 650                 655

Gln Glu Lys Val Thr Ala Leu Phe Thr Ser Thr Asn Pro Arg Gly Leu
            660                 665                 670

Lys Thr Asp Val Lys Asp Tyr His Ile Asp Gln Val Ser Asn Leu Val
        675                 680                 685

Glu Ser Leu Ser Asp Glu Phe Tyr Leu Asp Glu Lys Arg Glu Leu Phe
    690                 695                 700

Glu Ile Val Lys Tyr Ala Lys Gln Leu His Ile Glu Arg Asn Met
705                 710                 715

<210> 9
<211> 37
<212> DNA
<213> Artificial Sequence

<220>
<223> Description of Artificial Sequence: oligomer used
      for mutagenesis of the cry1Ba gene

<400> 9
ggacgcatcg tagtgcggac cgtacgaata cgattgg                                37


<210> 10
<211> 36
<212> DNA
<213> Artificial Sequence

<220>
<223> Description of Artificial Sequence: oligomer used
      for mutagenesis of the cry1Ia gene

<400> 10
ggacgcatcg tagtgcggac cgtacaaata caattg                                 36

## Description

### 1.0. Field of the invention

The present invention relates to hybrid toxin fragments, and toxins comprising them, derived from *Bacillus thuringiensis* insecticidal crystal proteins.

### 1.1. Description of the related art

*Bacillus thuringiensis* (hereinafter B.t.) is a gram-positive bacterium that produces insecticidal crystal proteins during sporulation. The crystal (Cry) proteins form a large (over 160 and growing) family of homologous proteins with unique specificities. Each protein is active against only one or a few insect species. Most reported proteins are active against lepidopterans, with a smaller number showing activity against diptera or coleoptera as reviewed in Schnepf *et al.,* 1998). While initially the Cry proteins were classified according to their activity against one of the abovementioned insect orders (Höfte and Whiteley, 1989), the more recent, now commonly accepted classification is based on amino acid homology (Crickmore *et al.,* 1998).

The mode of actions of crystal proteins has been partially elucidated. After ingestion by the insect larvae, crystals dissolve in the midgut environment, releasing the proteins as protoxins of 70-140 kDa. The solubilized protoxins are subsequently processed ("activated") by midgut proteases, resulting in a protease-resistant fragment of about 65 kDa, which is the active toxin. The toxin binds to receptors on epithelial cells of the insect midgut and penetrates the membrane. This eventually leads to lysis of the cells and death of the larvae.

The activity range of a particular delta-endotoxin is to a large extent determined by the occurrence of receptors on the midgut cells of the insect, although solubilization efficiency and proteolytic activition are also factors involved. The importance of binding to receptors is further examplified by the decrease in binding occuring in many instances of resistance to Cry proteins (Ferre *et al.,* 1995).

Structure determination by X-ray cristallography has shown that three different Cry proteins, and probably all Cry proteins, share a common three domain-structure ( Li *et al.,* 1991; Grochulski *et al.,* 1995; Morse *et al.,* 1998). If projected on Cry1 sequences, domain I runs from about amino acid residue 28 to 260, domain II from about 260 to 460 and domain III from about 460 to 620. Since the various toxins have different lengths, the borders of the domains can be defined only approximately. A person skilled in this art will be able to find the borders for the various toxins by comparing the amino acid sequences. The N-terminal domain I consists of 7 α-helices and is considered to be inserting (partially) into the target membrane, and forming part of the pore that eventually kills the insect gut epithelial cells. Both domain II and the C-terminal domain III are very variable, and have been shown to determine activity against specific insects. Although it is not yet clear how these domains individually or acting together may determine specificity, there is strong evidence that both can be involved in binding to (putative) receptors (Lee *et al.,* 1995; Dean *et al.,* 1996; de Maagd *et al.,* 1996b; de Maagd *et al.,* 1999).

Exchange of domain III between toxins by *in vivo* recombination of their encoding genes may not only alter specificity of a toxin, but can also result in a hybrid toxin with superior toxicity for certain insects (Bosch *et al.*, 1994; de Maagd *et al.,* 1 996a). Intl. Pat. Appl. Publ. No. WO 95/06730 discloses the construction of a hybrid delta-endotoxin consisting of domains I and II of Cry1E, and domain III and protoxin-specific fragment of Cry1C. In bioassays, this hybrid as purified after production in *E. coli* is active against *Manduca sexta, Spodoptera exigua,* and *Mamestra brassicae.* When expressed in and purified from a recombinant *Bacillus thuringiensis* strain, the 1E/1C-hybrid was 1.5 times as active as the most active natural toxin against *S. exigua,* Cry1C. The abovementioned patent application also describes a hybrid delta-endotoxin consisting of domains I and II of Cry1Ab, and domain III of Cry1C. When purified from a recombinant *Bacillus thuringiensis* strain, this Cry1 Ab/Cry1 C-hybrid was approximately 6.6 times as toxic as Cry1C (de Maagd *et al.,* 1 996a). Intl. Pat. Appl. Publ. No. WO 98/22595 discloses a number of hybrid toxins consisting of different combinations of fragments of Cry1Ab, Cry1Ac, Cry1C or Cry1F, of which some have improved acitivity against important pest larvae and/or an extended activity spectrum.

Producing succesfull hybrids is not easy. From the prior art it appears that only a very limited number of hybrids have an improved activity or a broader host-range specificity. In addition there are many possibilities for recombinantly-engineered crystal proteins, in view of the big number of different crystal proteins (more than 160). Further, the effect of the hybrids produced is not predictable.

### 2.0 Summary of the invention

Proteins of the Cry3 (Herrnstadt *et al.,* 1986; McPherson *et al.,* 1988), Cry7 (Lambert *et al.,* 1992) and Cry8 (Sato *et al.,* 1994) classes were found to be active against insects of the order Coleoptera (beetles). Cry3A is the singlemost active protein for the important potato pest Colorado potato beetle (CPB), *Leptinotarsa decemlineata* (Say).

It is known that resistance to the endotoxins may occur. Accordingly , resistance to Cry3A may occur in CPB in response to its exposure to Cry3A in transgenic potato. Actually, resistant CPB has already been disclosed. Therefore, it is desirable to have an alternative or replacement for Cry3A.

The present inventors have found specific hybrid crystal proteins which have a high activity against CPB and therefore can be used as a replacement for Cry3A. The hybrids of the invention comprise domains derived from Cry1la and Cry1Ba.

Cry1 proteins are generally active against lepidopterans (larvae of moths and butterflies). Cry1B and Cry1l have been shown to also have some activity against coleopterans, among which CPB, although their toxicity for CPB is much lower than that of Cry3A (Tailor *et al.*, 1992; Bradley *et al.*, 1995).

The toxicity of the hybrid crystal proteins of the invention against CPB, as compared to the parental proteins Cry1Ba and Cry1la, is surprisingly higher. Additionally a number of the hybrids of the invention have retained the high activity against some Lepidopterans, making them toxins with meaningful dual activity for Coleopterans and Lepidopterans.

According to the present invention there is provided a B.t. hybrid toxin fragment comprising structural domains I, II and III in this order starting from the N-terminal, wherein the domains are derived from at least two different Cry proteins, domain I is domain I of any of *B. t.* Cry protein or a part of said domain or a peptide substantially similar to said domain , domain II is domain II of Cry1la or a part of said domain or a peptide substantially similar to said domain, and domain III is domain III of Cry1Ba or a part of said domain or a peptide substantially similar to said domain. Preferred is a fragment which comprises domain I of Cry1la or Cry1Ba or a part of said domain or a peptide substantially similar to said domain, domain II of Cry1la or a part of said domain or a peptide substantially similar to said domain, and domain III of Cry1Ba or a part of said domain or a peptide substantially similar to said domain.

The term " or a peptide substantially similar to said domain" should be understood to mean a peptide having an amino acid sequence which is at least 85% similar to the sequence of the domain. It is preferred that the degree of similarity is at least 90%.

In the context of the present invention, two amino acid sequences with at least 85% or 90% similarity to each other have at least 85% or 90% identical or conservatively replaced amino acid residues in a like position when aligned optimally allowing for up to 6 gaps with the *proviso* that in respect of the gaps a total not more than 15 amino acid residues are affected. For the purpose of the present invention conservative replacements may be made between amino acids with the following groups:
1. Serine and Threonine;
2. Glutamic acid and Aspartic acid;
3. Arginine and Lysine
4. Asparagine and Glutamine
5. Isoleucine, Leucine, Valine, and Methionine;
6. Phenylalanine, Tyrosine, and Tryptophan
7. Alanine and Glycine
By "or a part of said domain" is meant a peptide comprised by the said domain and having at least 80% of the consecutive sequence thereof.

It is most particularly preferred that the toxin fragment according to the invention comprises either:
1. An amino acid sequence from about amino acid 20 to about amino acid 641 in SEQ ID NO:2 or
2. An amino acid sequence from about amino acid 20 to about amino acid 632 in SEQ ID NO:4.

The invention also includes a hybrid toxin comprising the above disclosed fragment or a toxin at least 85% similar to such a hybrid toxin which has substantially similar insecticidal activity.

The hybrid toxin comprises the three structural domains as defined above and generally may comprise a pro-toxin segment at the carboxyl end, which is not toxic and is thought to be important for crystal formation. SEQ ID NO:2 and 4 comprise such pro-toxin segment. Further, the hybrid toxin may comprise a lead sequence, being amino acids 1-19 in SEQ ID NO:2 and 4.

The invention still further includes pure proteins which are at least 90% identical to the toxin fragments or hybrid toxins according to the invention.

The invention still further includes recombinant DNA comprising a sequence encoding a protein having an amino acid sequence of the above disclosed toxins or fragments thereof.

In a preferred embodiment the invention provides recombinant DNA comprising the sequence as shown in SEQ ID NO:1 or 3 or DNA similar thereto encoding a substantially similar protein.

In a more preferred embodiment the invention provides recombinant DNA comprising the sequence from about nucleotide 170 to about 1929 in SEQ ID NO:1 or from about nucleotide 147 to about 1896 in SEQ ID NO:3.

By similar DNA is meant a test sequence which is capable of hybridizing to the inventive recombinant sequence. When the test and inventive sequences are double stranded the nucleic acid constituting the test sequence preferably has a Tₘ within 20°C of that of the inventive sequence. In the case that the test and inventive sequences are mixed together and denantured simultaneously, The Tₘ values of the sequences are preferably within 10°C of each other. More preferably the hybridization is performed under stringent conditions, with either the test or inventive DNA preferaby being supported. Thus either a denatured test or inventive sequence is preferably first bound to a support and hybridization is effected for a specified period of time at a temperature of between 50 and 70 °C in double strength citrate buffered saline containing 0.1 % SDS followed by rinsing of the support at the same temperature but with a buffer having a reduced SSC concentration. Depending upon the degree of stringency required, and thus the degree of similarity of the sequences, such reduced concentration buffers are typically single strength SSC containing 0.1 % SDS. Sequences having the greatest degree of similarity are those the hybridization of which is least affected by washing in buffers of reduced concentration. It is most preferred that the test and inventive sequences are so similar that the hybridization between them is substantially unaffected by washing or incubation in one tenth strength sodium citrate buffer containing 0.1% SDS.

The recombinant DNA may further encode a protein having herbicide resistance, plant growth-promoting, anti-fungal , anti-bacterial, anti-viral and/or anti-nematode properties. In the case that the DNA is to be introduced into a heterologous organism it may be modified to remove known mRNA instability motifs (such as AT rich regions) and polyadenylation signals, and/or codons which are preferred by the organism into which the recombinant DNA is to be inserted may be used so that expression of the thus modified DNA in the said organism yields substantially similar protein to that obtained by expression of the unmodified recombinant DNA in the organism in which the protein components of the hybrid toxin or toxin fragments are endogenous.

The invention still further includes a DNA sequence which is complementary to one which hybridizes under stringent conditions with the recombinant DNA according to the invention.

Also included in the present invention are: a vector containing such a recombinant (or complementary thereto) DNA sequence; a plant or micro-organism which includes, and enables expression of such DNA; plants transformed with such DNA; the progeny of such plants which contain the DNA stably incorporated and hereditable in a Mendelian manner, an/or the seeds of such plants and such progeny. The invention still further includes protein derived from expression of the said DNA, and insecticidal protein produced by expression of the recombinant DNA within plants transformed therewith.

The invention still further includes an insecticidal composition containing one or more of the toxin fragments or toxins comprising them according to the invention; a process for combatting insects which comprises exposing them to such fragments or toxins or compositions, and an extraction process for obtaining insecticidal proteins from organic material containing them comprising submitting the material to maceration and solvent extraction.

The invention will be further apparent from the following description, which describes the production of Bt hybrid toxin fragments according to the invention, taken in conjunction with the associated drawings and sequence listings.

A person skilled in the art may use different methods to construct the hybrid toxin genes of the invention. Domain encoding regions may be exchanged between two homologous, though different genes by exchanging fragments through restriction enzyme digestion and subsequent ligation, if the same restriction enzyme recognition sites are present at the same position in the two genes. If suitable restriction enzyme recognition sites are not available, new sites can be created at homologous positions in the two genes by various methods of site-directed mutagenesis, as described in the examples below for construction of a common Rsrll-site using DNA oligomers SEQ ID: 9 and SEQ ID: 10.

Alternatively, fragments that are to be combined in a hybrid toxin gene may be produced by PCR-mediated amplification using the original genes as templates, taking care that the ends of the fragments contain compatible restriction enzyme digestion sites. Furthermore, a hybrid toxin encoding DNA fragment may be produced *by in vitro* recombination between overlapping, partially homologous DNA fragments as performed in so-called DNA shuffling experiment (Crameri et al, 1998; Zhao et al., 1998). Also, hybrid toxin encoding DNA fragments may be produced by *in vivo* recombination between two homologous DNA fragments that have been cloned in tandem on a single plasmid (Bosch et al., 1994), followed by screening for the desired recombination events.

### 3.0 Brief description of the drawings

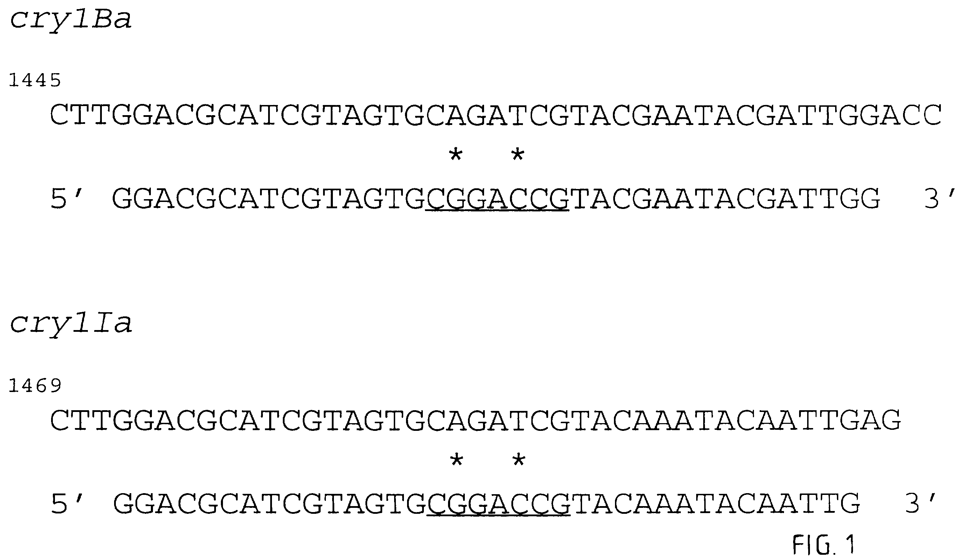

**Figure 1** shows the sequence of the relevant part of the *cry1Ba* (top) and *cry1la* (bottom) genes, respectively, aligned with the respective oligomers ( SEQ ID NO:9 and 10) used for mutagenesis in order to produce a common *Rsr*ll restriction enzyme recognition site in both genes. Mutated nucleotides are indicated by asterisks and the newly produced *Rsr*ll recognition sites are underlined.

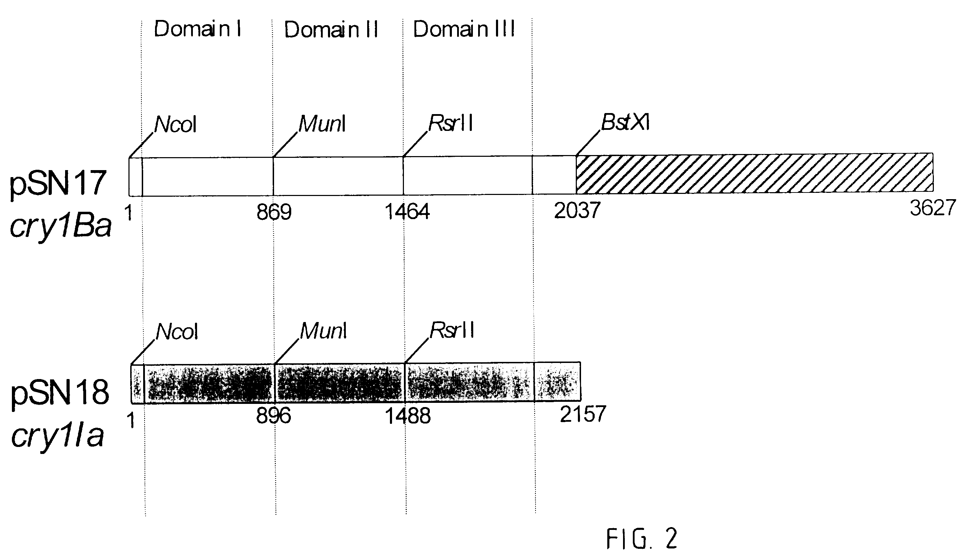

**Figure 2** shows both the *cry1la* gene (solid bar) as well as the *cry1Ba* fragment (open bar) presented as they occur in expression vectors pSN18 and pSN17, respectively. Both genes are inserted in the pKK233-2 derived expression vector for *E. coli,* pBD12. For construction of pSN18 the 3' part of the *cry1Ba* gene, encoding the C-terminal part of the protoxin-specific fragment, was replaced by the corresponding part of the *cry1Ca* gene (base pairs 2038-3627; dashed bar). The positions of the common *Nco*l and *Mun*l restriction sites, as well as of the *Rsr*ll sites derived by mutagenesis, and the *BstX*l-site used for exchange of the 3'end of the *cry1Ba* gene are indicated, with nucleotide position numbers below the respective genes.

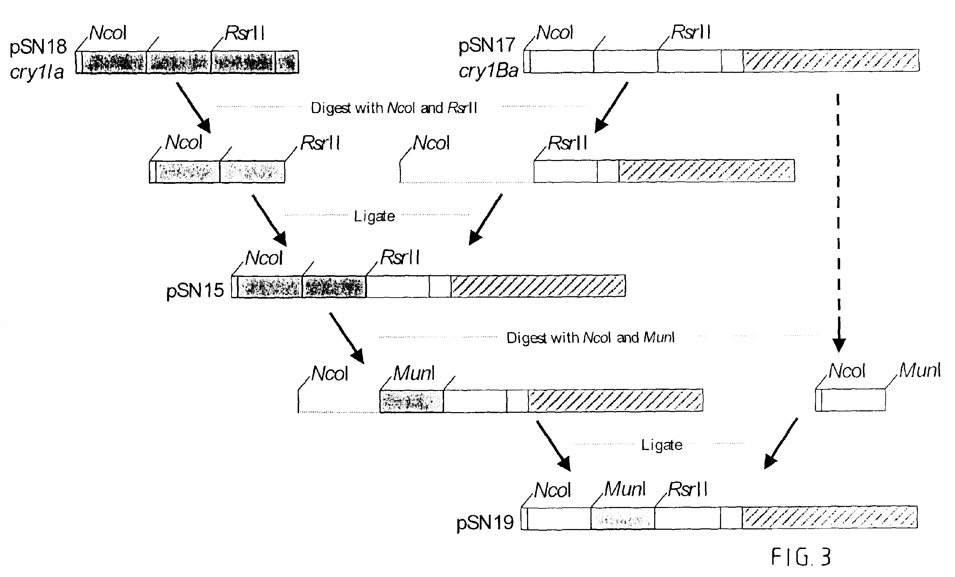

**Figure 3** shows in a scheme the construction of hybrid gene SN15 from *cry1Ba* (pSN17) and *cry1la* (pSN18), and the subsequent construction of hybrid gene SN19 from *cry1Ba* (pSN17) and pSN15.

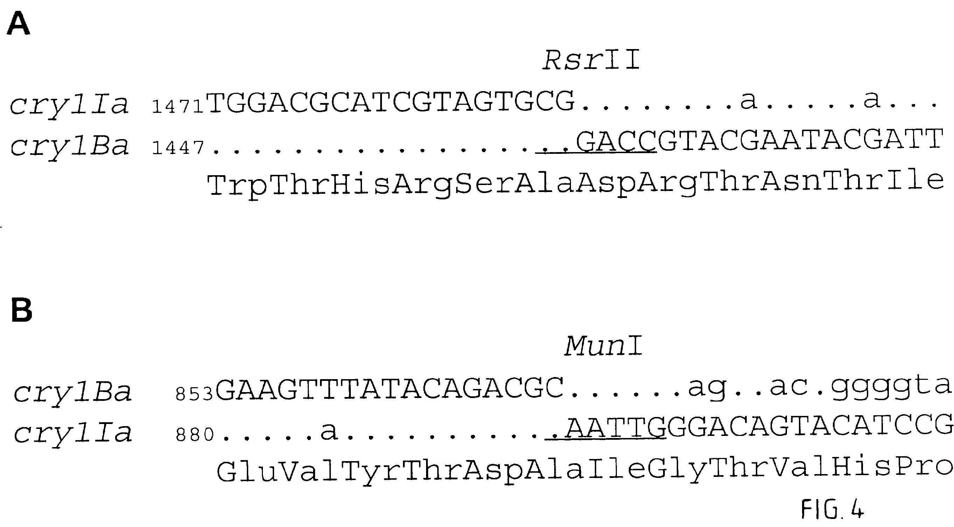

**Figure 4** shows the alignment of *cry1Ba* and *cry1la* nucleotide sequences, as present in plasmids pSN17 and pSN18, respectively, around the junctions between the domain II and III (A) and between domain I and II (B). Nucleotide sequences as present in the hybrid genes of SN15 and pSN19 (A) or of pSN19 alone (B) are given in capitals, while identical nucleotides in the DNA that is not present in SN15 and SN19 is represented by dots. The position of the common *Rsr*ll and *Mun*l recognition sites are underlined. The site of crossover in the hybrids is represented by the shift of the fully written out nucleotide sequence from top strand to bottom strand in this recognition sites. The amino acid sequence of the resulting hybrid proteins is given in three-letter code below each alignment.

### 4.0 Brief description of the sequence identifiers

**SEQ ID NO:1** shows the nucleotide sequence of the hybrid protoxin gene SN15.

**SEQ ID NO:2** shows the amino acid sequence of the protein encoded by the gene SN15 shown in SEQ ID NO:1.

**SEQ ID NO:3** shows the nucleotide sequence of the hybrid protoxin gene SN19.

**SEQ ID NO:4** shows the amino acid sequence of the protein encoded by the gene SN19 shown in SEQ ID NO:3.

**SEQ ID NO:5** shows the nucleotide sequence of the modified *cry1Ba* gene as used in expression vector pSN17.

**SEQ ID NO:6** shows the amino acid sequence of the protein encoded by the *cry1Ba* gene shown in SEQ ID NO:5.

**SEQ ID NO:7** shows the nucleotide sequence of the modified *cry1la* gene as used in expression vector pSN18.

**SEQ ID NO:8** shows the amino acid sequence of the protein encoded by the *cry1la* gene shown in SEQ ID NO:7.

**SEQ ID NO:9** shows the nucleotide sequence of the oligomer used for mutagenesis of the *cry1Ba* gene.

**SEQ ID NO:10** shows the nucleotide sequence of the oligomer used for mutagenesis of the *cry1la* gene.

### 5.0 Examples

### 5.1 DNA manipulations

All recombinant DNA techniques are as described by Ausubel et al. (1997). Mutagenesis, restriction enzyme digestion and ligation are performed according the instructions of the manufacturers. DNA sequencing is performed by the dideoxytriphosphate methode with fluorescent dyes attached to the dideoxynucleotides. Analysis is automated by using an Applied Biosystems 370A nucleotide sequence analyzer.

### 5.2 Expression vectors

All Cry protein expression vectors are based on pBD12, a derivative of pKK233-2 (Bosch *et al.,* 1994). For expression of Cry3Aa protein, the full *cry3Aa* gene is cloned into pBD12, giving expression plasmid pMH10. For cloning purposes both *cry1Ba* as well as *cry1la* are mutagenized in order to contain a *Nco*l-site overlapping with the start codon. For production of Cry1Ba protein, a *Ncol-BstX*l (bases 1-1977) fragment of *cry1Ca* in pBD150 (Bosch *et al.*, 1994) is replaced by the corresponding fragment of *cry1Ba* (bases 1-2037), resulting in *cry1Ba* expression vector pMH19. pMH19 therefor contains the 5'active toxin encoding part of Cry1Ba and 3' protoxin specific part of Cry1Ca. Cry1la expression vector pBD172 contains the full *cry1la* gene with the *Spe*l-site (base 2180) fused to the Spel-site in the polylinker of pBluescript SK⁺.

### 5.3 Mutagenesis of cry1Ba and cry1la

In order to be able to directly exchange the domain III encoding regions between *cry1Ba* and *cry1la,* a new common restriction enzyme recognition site is made in both genes by site directed mutagenesis. Complementary mutagenic oligonucleotide pairs are used to create unique Rsrll-sites at positions 1464 and 1488 of *cry1Ba* (pMH19) and *cry1la* (pBD172), respectively (see Fig. 1), using the using the QuickChange™ kit (Stratagene). These mutations do not change the encoded proteins. This results in two new expression plasmids, pSN17 (Cry1Ba, Seq. ID NO: 5) and pSN18 (Cry1la, Seq. ID NO: 7), respectively (Fig. 2). The unique *Rsr*ll restriction sites at the border of the domain II and domain III encoding regions, together with the common *Mun*l-sites at the border between the domain I and domain II encoding regions allowed simple swapping of domain encoding regions between the two genes.

### 5.4 Construction of hybrid toxins

Fig. 3 schematically shows the construction of two novel hybrid toxins. Both pSN18 and pSN17 are digested with *Nco*l and *Rsr*ll*.* Subsequently, the 1488 base fragment of pSN18, containing the domain I and II encoding fragment of *cry1la* is ligated into the corresponding sites of the pSN17-derived fragment containing the 3'portion of *cry1Ba.* This results in plasmid pSN15 encoding a 1la/1la/1Ba-hybrid (Seq. ID NO:1). The nucleotide sequence of the cross-over region with the encoded amino acid sequence is shown in Fig. 4A. Subsequently a *Nco*l-*Mun*l (base 1-896) fragment encoding domain I of Cry1la from pSN15 is replaced by the corresponding fragment encoding domain I of Cry1Ba, derived from pSN17. This results in 1Ba/1la/1 Ba-hybrid encoding plasmid pSN19 (Seq. ID. NO:3). The nucleotide sequence of the crossover region with the encoded amino acid sequence is shown in Fig. 4B.

### 5.5 Protein isolation and insect bioassays.

For large-scale production, all parental and hybrid protoxins are expressed in *E. coli* strain XL-1 and extracted as described earlier (Bosch *et al.,* 1994). Solubilized protoxins are dialyzed overnight in 25 mM NaHC₃, 100 mM NaCI, pH10. Protein concentrations are estimated by SDS-PAGE (sodium dodecylsulphate polyacrylamide gel electrophoresis). To test toxicity to Colorado potato beetle (CPB), leaflets of greenhouse grown potato culitivar Desiree are dipped in toxin dilutions in water containing 0.01 % Tween-20. After drying of the leaves to the air they are transferred to petri dishes and 10 neonate CPB larvae are placed on each leaf. After incubation for two days at 28°C, the leaves are replaced by fresh leaves dipped in identical protoxin dilutions. Mortality is scored after 4 days. LC₅₀ (concentration with 50% mortality) and 95% fiducial limits are determined by Probit analysis of results from three or more independent experiments, using the PoloPC computer program (Russel *et al.,* 1977).

### 5.6 Toxicity of wild type and hybrid proteins to Colorado potato beetle

Table 1 shows the toxicity of the hybrid proteins SN15 and SN19 against Colorado potato beetle (CPB), as compared to the parental proteins Cry1Ba and Cry1la, and to the most CPB-active natural toxin available, Cry3A. 1la/1la/1Ba-hybrid SN15 shows slightly higher toxicity (lower LC50) than its best parental toxin, Cry1la, on a per weight basis. When considering that the molecular weight of the SN15 protein is considerably larger than that of Cry1la, it follows that SN15 performs even better on a per mol basis. 1Ba/1la/1Ba-hybrid protein SN19 is even considerably more toxic than SN15.

**Table 1.**

| Toxicity of wild type and hybrid protoxins to Colorado potato beetles. | | | | |
|---|---|---|---|---|
| Protoxin | LC₅₀^{a} | 95% fiducial limits ^{a} | MW^{b} | Relative toxicity^{c} |
| | | | | |
| MH10 (Cry3A) | 1.8 | 1.4-2.5 | 74.0 | 100 |
| SN17(Cry1Ba) | 142 | 105-198 | 137.4 | 1 |
| SN18(Cry1la) | 34 | 23-47 | 81.3 | 6 |
| SN15 | 22 | 14-35 | 138.0 | 15 |
| SN19 | 8 | 5-11 | 137.2 | 42 |

| | | | | |
|---|---|---|---|---|
| ^{a}Concentration in microgram per milliliter of dipping solution. LC_{5O}: concentration which leads to 50% mortality; | | | | |
| ^{b}Approximate molecular weight in kiloDaltons. | | | | |
| ^{c}Relative toxicity on molar basis in percents, with toxicity of Cry3A set at 100%. | | | | |

Although the present invention has been particularly described for the production of SN15 and SN19 hybrid toxins, the skilled scientist will appreciate that other hybrid toxins with improved insecticidal characteristics may be produced according to the invention. Hybrid toxins containing the combination of Cry1la domain II and Cry1Ba domain III, with various combinations of domain I and C-terminal extensions may be made. Moreover, the gene encoding SN15, SN19 and/or other hybrids may be transferred into strains of B.t. and/or integrated into the chromosome of strains of B.t., of other bacteria or be introduced into plant genomes to provide for in situ insecticidal activity within the plant per se. In this regard, it is particularly preferred that the recombinant DNA encoding the toxins is modified in that sequences which are detrimental to high level expression in plants are removed and in that codons which are preferred by the plant are used. This should lead to production in the plant of a substantially similar protein to that obtained by expression of the unmodified recombinant DNA in the organism in which the protein components of the hybrid toxins or toxin fragments are endogenous.

### 6.0 References

The following references, to the extent that they provide exemplary procedural or other details supplementary to those set forth herein, are specifically incorporated herein by reference.
Intl. Pat. Appl. Publ. No. WO 95/06730, published 9 March 1995
Intl. Pat. Appl. Publ. No. WO 98/22595, published 28 May 1998
Ausubel, F. *et al.* (1997) *Current Protocols in Molecular Biology.* John Wiley & Sons.
Bosch, D., Schipper, B., van der Kleij, H., de Maagd, R. and Stiekema, W. (1994) Recombinant *Bacillus thuringiensis* crystal proteins with new properties: possibilities for resistance management. *Bio/technology,* **12**, 915-918.
Bradley, D., Harkey, M.A., Kim, M.K., Biever, K.D. and Bauer, L.S. (1995) The insectidal CrylB crystal protein of *Bacillus thuringiensis* ssp. *thuringiensis* has dual specificity to Coleopteran and Lepidopteran larvae. *Journal of Invertebrate Pathology,* **65**, 162-173.
Crameri, A., S. A. Raillard, E. Bermudez, and W. P. C. Stemmer. 1998. DNA shuffling of a family of genes from diverse species accelerates directed evolution. Nature. 391(6664):288-291.
Crickmore, N., Zeigler, D.R., Feitelson, J., Schnepf, E., VanRie, J., Lereclus, D., Baum, J. and Dean, D.H. (1998) Revision of the nomenclature for the Bacillus thuringiensis pesticidal crystal proteins. *Microbiology and molecular biology reviews,* **62**, 807&.
de Maagd, R.A., Bakker, P.L., Masson, L., Adang, M.J., Sangadala, S., Stiekema, W. and Bosch, D. (1999) Domain III of the *Bacillus thuringiensis* delta-endotoxin Cry1Ac is involved in binding to Manduca sexta brush border membranes and to its purified aminopeptidase N. *Molecular microbiology,* **31**,463-471.
de Maagd, R.A., Kwa, M.S.G., van der Klei, H., Yamamoto, T., Schipper, B., Vlak, J.M., Stiekema, W.J. and Bosch, D. (1996a) Domain III substitution in *Bacillus thuringiensis* delta-endotoxin CrylA(b) results in superior toxicity for *Spodoptera exigua* and altered membrane protein recognition. *Applied & Environmental Microbiology,* **62**, 1537-1543.
de Maagd, R.A., van der Klei, H., Bakker, P.L., Stiekema, W.J. and Bosch, D. (1996b) Different domains of *Bacillus thuringiensis* delta-endotoxins can bind to insect midgut membrane proteins on ligand blots. *Applied & Environmental Microbiology,* **62**, 2753-2757.
Dean, D.H., Rajamohan, F., Lee, M.K., Wu, S.J., Chen, X.J., Alcantara, E. and Hussain, S.R. (1996) Probing the mechanism of action of *Bacillus thuringiensis* insecticidal proteins by site directed mutagenesis: a minireview. *Gene,* **179,** 111-117.
Ferre, J., Escriche, B., Bel, Y. and van Rie, J. (1995) Biochemistry and genetics of insect resistance to *Bacillus thuringiensis* insecticidal crystal proteins. *FEMS Microbiology Lett.,* **132,** 1-7.
Grochulski, P., Masson, L., Borisova, S., Pusztai-Carey, M., Schwartz, J.L., Brousseau, R. and Cygler, M. (1995) *Bacillus thuringiensis* CrylA(a) insecticidal toxin - crystal structure and channel formation. *Journal of Molecular Biology,* **254**, 447-464.
Herrnstadt, C., Soares, G.G., Wilcox, E.R. and Edwards, D.L. (1986) A new strain of Bacillus thuringiensis with activity against coleopteran insects. *Biotechnology,* **4**, 305-308.
Höfte, H. and Whiteley, H.R. (1989) Insecticidal crystal proteins of *Bacillus thuringiensis. Microbiol. Rev.,* **53**, 242-255.
Lambert, B., Hofte, H., Annys, K., Jansens, S., Soetaert, P. and Peferoen, M. (1992) Novel *Bacillus thuringiensis* insecticidal crystal protein with a silent activity against coleopteran larvae. *Applied And Environmental Microbiology,* **58**, 2536-2542.
Lee, M.K., Young, B.A. and Dean, D.H. (1995) Domain III exchanges of *Bacillus thuringiensis* Cry1A toxins affect binding to different Gyspy Moth midgut receptors. *Biochemical and Biophysical Research Communications,* **216**, 306-312.
Li, J., Carroll, J. and Ellar, D.J. (1991) Crystal structure of insectidicidal delta-endotoxin from *Bacillus thuringiensis* at 2.5 A resolution. *Nature (London),* **353**, 815-821.
McPherson, S., Perlak, F., Fuchs, R., Marrone, P., Lavrik, P. and Fischhoff, D. (1988) Characterization of the coleopteran-specific protein gene of *Bacillus thuringiensis* var. *tenebrionis. Biotechnology,* **6***,* 61-66.
Morse, R.J., Powell, G., Ramalingam, V., Yamamoto, T. and Stroud, R.M. (1998) Crystal structure of Cry2Aa from *Bacillus thuringiensis* at 2.2 Angstroms: structural basis of dual specificity. In lizuka, T. (ed.) *Vllth International Colloquium on Invertebrate Pathology and Microbial Control and IVth International Conference on Bacillus thringiensis.* Sapporo, pp. 1-2.
Russel, R.M., Robertson, J.L. and Savin, N.E. (1977) POLO: a new computer program for Probit analysis. *ESA Bulletin,* **23**, 209-213.
Sato, R., Takeuchi, K., Ogiwara, K., Minami, M., Kaji, Y., Suzuki, N., Hori, H. and Asano, S. (1994) Cloning, heterologous expression, and localization of a novel crystal protein gene from bacillus-thuringiensis serovar japonensis strain buibui toxic to scarabaeid insects. *Curr Microbiol,* **28**, 15-19.
Schnepf, E., Crickmore, N., van Rie, J., Lereclus, D., Baum, J., Feitelson, J., Zeigler, D.R. and Dean, D.H. (1998) *Bacillus thuringiensis* and its pesticidal crystal proteins. *Microbiology and molecular biology reviews,* **62**, 775-806.
Tailor, R., Tippet, J., Gibb, G., Pells, S., Pike, D., Jordan, L. and Ely, S. (1992) Identification and characterization of a novel *Bacillus thuringiensis* delta-endotoxin entomocidal to coleopteran and lepidopteran larvae. *Mol. Microbiol.,* **6**, 1211-1217.
Zhao, H. M., L. Giver, Z. X. Shao, J. A. Affholter, and F. H. Arnold. 1998. Molecular evolution by staggered extension process (StEP) in vitro recombination. Nature biotechnology. **16**(3):258-261.

## Claims

1. *Bacillus thuringiensis* hybrid toxin fragment comprising structural domains I, II and III in this order starting from the N-terminal, wherein the domains are derived from at least two different Cry proteins, domain I is domain I of any *Bacillus thuringiensis* Cry protein or a part of said domain or a peptide substantially similar to said domain, domain II is domain II of Cry1la or a part of said domain or a peptide substantially similar to said domain, and domain III is domain III of Cry1Ba or a part of said domain or a peptide substantially similar to said domain.

2. Toxin fragment according to claim 1, wherein domain I is domain I of Cry1la or Cry1Ba or a part of said domain or a peptide substantially similar to said domain .

3. Toxin fragment according to claim 1 or 2 comprising an amino acid sequence from about amino acid 20 to about amino acid 641 as shown in SEQ ID NO:2 or an amino acid sequence from about amino acid 20 to about amino acid 632 as shown in SEQ ID NO:4.

4. A hybrid toxin comprising the fragment of any one of the preceding claims , or a toxin at least 85% similar to such a hybrid toxin which has substantially similar insecticidal activity.

5. A toxin according to claim 4 comprising an amino acid sequence as shown in SEQ ID NO:2 or SEQ ID NO:4.

6. Pure proteins which are at least 90% identical to the toxin fragments or hybrid toxins of any of claims 1-5.

7. Recombinant DNA comprising a sequence encoding a protein having an amino acid sequence of the proteins as claimed in any one of claims 1-6.

8. Recombinant DNA according to claim 7 comprising the sequence as shown in SEQ ID N0:1 or 3 or DNA similar thereto encoding a substantially similar protein.

9. Recombinant DNA according to claim 7 comprising the nucleotide sequence from about nucleotide 170 to about 1929 in SEQ ID N0:1 or from about 147 to about 1896 in SEQ ID NO:3.

10. Recombinant DNA according to any of claims 7-9, which further encodes a protein having herbicide resistance, plant growth-promoting, anti-fungal, anti-bacterial, antiviral and/or anti-nematode properties.

11. Recombinant DNA according to any one of claims 7 to 10 which is modified in that known mRNA instability motifs or polyadenylation signals are removed and/or codons which are preferred by the organism into which the recombinant DNA is to be inserted are used so that expression of the thus modified DNA in the said organism yields substantially similar protein to that obtained by expression of the unmodified recombinant DNA in the organism in which the protein components of the hybrid toxin or toxin fragments are endogenous.

12. A DNA sequence which is complementary to one which hybridizes under stringent conditions with the DNA of any one of claims 7 to 11.

13. A vector containing a DNA sequence as claimed in any one of claims 7 to 12.

14. A plant or micro-organism which includes, and enables expression of, the DNA of any one of claims 7-12 or the vector of claim 13.

15. Plants transformed with recombinant DNA as claimed in any one of claims 7 to 12, the progeny of such plants which contain the DNA stably incorporated and hereditable in a Mendelian manner, and/or the seeds of such plants and such progeny.

16. Protein derived from expression of the DNA as claimed in any one of claims 7 to 12 and insecticidal protein produced by expression of the recombinant DNA within plants as claimed in claim 15.

17. An insecticidal composition containing one or more of the proteins as claimed in any one of claims 1-6 and 16.

18. A process for combatting insects which comprises exposing them to proteins or compositions as claimed in any one of claims 1-6, 16 and 17 or the micro-organism of claim 14.

19. An extraction process for obtaining insecticidal proteins, as claimed in any one of claims 1-6 or claim 16, from organic material containing them comprising submitting the material to maceration and solvent extraction, characterized in that the material is a microorganism.
